# EUROPEAN PATENT APPLICATION

(11) **EP 1 586 903 A1**
(43) Date of publication of application: **19.10.2005**
(21) Application number: 05006874.1
(22) Date of filing: 30.03.2005
(51) Int. Cl.: G01N 33/574

(54) **Method for screening cervical cancer**

(30) Priority: 30.03.2004 JP 2004097460
(71) Applicant: SYSMEX CORPORATION, Kobe-shi, Hyogo 651-0073 (JP)
(72) Inventor: Nakano, Koichi, c/o Sysmex Corporation, Kobe-shi, Hyogo 651-0073 (JP); Ishisaka, Masaki, c/o Sysmex Corporation, Kobe-shi, Hyogo 651-0073 (JP); Kishi, Kazuki, c/o Sysmex Corporation, Kobe-shi, Hyogo 651-0073 (JP); Watanabe, Miharu, c/o Sysmex Corporation, Kobe-shi, Hyogo 651-0073 (JP); Imura, Yasuyuki, c/o Sysmex Corporation, Kobe-shi, Hyogo 651-0073 (JP)
(74) Representative: HOFFMANN EITLE

(57) **Abstract**

The invention provides a reagent for diagnosing cervical cancer, which can not only detect the presence or absence of cervical cancer but can also distinguish squamous cell carcinoma and adenocarcinoma from each other, a method for screening cervical cancer by using the reagent, particularly a screening method capable high speed processing by utilizing flow cytometry.

The reagent comprises a first labeled antibody reacting with gland cells, a second labeled antibody reacting with adenocarcinoma cells and a third labeled antibody reacting with atypical cervical squamous cells, the antibodies being labeled with mutually distinguishable labels respectively. Preferably, at least one selected from the group consisting of MUC1 antibody, cytokeratin 7 antibody, and cytokeratin 18 antibody is used as the first labeled antibody, at least one selected from the group consisting of cytokeratin 8 antibody and HIK1083 antibody is used as the second labeled antibody, and at least one member selected from the group consisting of NMP179 antibody, p16^{INK4A} antibody, Ki-67 antibody, p53 antibody, p21 antibody, EMA antibody, CEA antibody and MIB-1 antibody is used as the third labeled antibody.

## Description

### BACKGROUND OF THE INVENTION

### 1. Field of the Invention

The present invention relates to a reagent for diagnosing cervical cancer, which can detect the presence or absence of squamous cell carcinoma, its precancerous state, adenocarcinoma and its precancerous state in a sample of cervical cell group collected from the living body, a method for screening cervical cancer by using the diagnostic reagent, and an automatic diagnostic method of automatically judging adenocarcinoma and squamous cell carcinoma.

### 2. Description of the Related Art

As a method of early finding of cervical cancer, cellular diagnosis is utilized effectively in medical examinations.

Cellular diagnosis for cervical cancer is conduced by scrubbing a cervical surface with a cotton swab or a scrubber, immediately smearing the scrubbed cells on a slide glass to prepare a sample and observing the sample under a microscope or the like.
The diagnosis of cells by observing the form of the cells under a microscope is conducted for each sample by a cytotechnologist, and there is need for improvements in accuracy and processing speed.

In recent years, an apparatus for judging the presence or absence of cancer cells by automatically examining cell samples is commercially available. In this automatically judging apparatus, a sample containing cervical cells is smeared on a slide glass to prepare a smear sample, and nuclei and cytoplasm in the sample are stained with Papanicolaou stain, and the presence or absence of cancer cells therein is judged from information on the processed image of the sample cell. However, the performance of this apparatus for automatically judging cells is that the removal efficiency of normal samples is 25%, and the processing speed is about 8 to 10 samples/hour. Such accuracy and processing speed are not satisfactory for those who judge the presence or absence of cancer in medical examinations.

On the other hand, there is a cellular diagnostic method of judging the presence or absence of cancer cells by detecting a marker specific to cancer cells, instead of the observation of cellular form.

For example, US Patent No. 5,858,683 (Keesee *et al*.) has proposed an immunoassay wherein a cervical cancer-related protein and an antibody recognizing the same are used as markers specific to cervical cancer and its precancerous state. As the antibody recognizing a cervical cancer-related protein, NMP 179 antibody is known.

US Patent Application No. 2002/0106685 (Henning *et al*.) has proposed a method of automatically detecting tumor cells and their precursor cells not only in a cervical smear but also in a sample of individually dispersed cells. This method is a method of detecting the presence or absence of cancer cells, which comprises using a reagent consisting of a fluorescence-labeled antibody or nucleic acid probe reacting specifically with two or more kinds of markers on cancer cells to automatically measure the presence or absence of a fluorescence signal of the reagent bound to the markers. The markers include her2/neu, p16, p53, MN, mdm-2, bcl-2, and EGF receptors, as well as HPV6, 11, 16, 18, 30, 31, 33, 34, 35, 45, 51 and 52.

The cervical cancer is classified roughly into squamous cell carcinoma, adenocarcinoma, and their precancerous state, and there is demand in recent years for a screening method capable of diagnosing whether cancer cells detected by medical examinations are those of squamous cell carcinoma or adenocarcinoma.

However, the markers used in US Patent Application No. 2002/0106685 supra are markers for general cancer cells, and thus the presence or absence of cancer cells can be judged, but the type of cervical cancer cannot be identified. The marker disclosed in US Patent No. 5,858,683 supra is a marker specific to cervical cancer, but it is a marker common to squamous cell carcinoma and adenocarcinoma, so information for identifying the type of cancer cannot be obtained.

NMP179 antibody is reported to react with a part of normal cells (Acta Cytologica, Volume 43, Number 6/November-December 1999:1015-1022), and its performance cannot be satisfactory.

### SUMMARY OF THE INVENTION

The present invention was made in view of the circumstances described above, and the object of the present invention is to provide a reagent for diagnosing cervical cancer, which can not only detect the presence or absence of cervical cancer but can also distinguish squamous cell carcinoma and adenocarcinoma from each other, a method for screening cervical cancer by using the reagent, particularly a method for screening cervical cancer which is capable of high speed processing by utilizing flow cytometry.

The method for screening cervical cancer according to a first aspect of the present invention is a method comprising the steps of preparing a measurement sample by reacting, with cervical cells, a first labeled antibody reacting with gland cells, a second labeled antibody reacting with adenocarcinoma cells and a third labeled antibody reacting with atypical cervical squamous cells, the antibodies being labeled with mutually distinguishable labels respectively, detecting the labels derived from the respective labeled antibodies bound to the cervical cells, and judging the presence or absence of adenocarcinoma cells and/or atypical squamous cells, on the basis of the detected labels.

The method for screening cervical cancer according to a second aspect of the present invention is a method comprising the steps of preparing a measurement sample by reacting cervical cells with a first fluorescence-labeled antibody reacting with gland cells, a second fluorescence-labeled antibody reacting with adenocarcinoma cells and a third fluorescence-labeled antibody reacting with atypical cervical squamous cells, the antibodies being labeled with mutually distinguishable fluorescences respectively, irradiating the measurement sample with an exciting light, detecting the fluorescence emitted by the measurement sample, and judging the presence or absence of adenocarcinoma cells and/or atypical squamous cells, on the basis of the detected fluorescence.

The diagnostic reagent for cervical cancer according to the present invention comprises a first fluorescence-labeled antibody reacting with gland cells, a second fluorescence-labeled antibody reacting with adenocarcinoma cells and a third fluorescence-labeled antibody reacting with atypical cervical squamous cells, the antibodies being labeled with mutually distinguishable fluorescences respectively.

The method of automatically diagnosing cervical cancer according to the present invention is a method wherein in the screening method according to the first and second aspects of the present invention, the presence or absence of adenocarcinoma cells and/or atypical squamous cells is automatically judged, and when the ratio of cells judged to be adenocarcinoma cells and/or atypical squamous cells to the cell population contained in the measurement sample is higher than a predetermined value, the cervical cancer is judged to be adenocarcinoma or squamous cell carcinoma.

The method for screening cervical cancer according to a third aspect of the present invention is a method comprising the steps of preparing a measurement sample by reacting cervical cells with an anti-gland cell labeled antibody reacting with gland cells and an anti-atypical squamous cell antibody reacting with atypical cervical squamous cells, the antibodies being labeled with mutually distinguishable labels respectively, detecting labels derived respectively from the respective labeled antibodies bound to the cervical cells, and judging the presence or absence of atypical squamous cells, on the basis of the detected labels.

The apparatus for screening cervical cells according to the present invention is an apparatus comprising a flow cell for introducing a measurement sample prepared by reacting cervical cells with a first fluorescence-labeled antibody reacting with gland cells, a second fluorescence-labeled antibody reacting with adenocarcinoma cells and a third fluorescence-labeled antibody reacting with atypical cervical squamous cells, the antibodies being labeled with mutually distinguishable fluorescent substances respectively, a light source for irradiating the cells in the measurement sample flowing in the flow cell with an exciting light, a first fluorescence detector for detecting the fluorescence derived form the first fluorescence-labeled antibody emitted by the cells irradiated with an exciting light, a second fluorescence detector for detecting the fluorescence derived form the second fluorescence-labeled antibody emitted by the cells irradiated with an exciting light, a third fluorescence detector for detecting the fluorescence derived form the third fluorescence-labeled antibody emitted by the cells irradiated with an exciting light, and an analysis unit for analyzing the detected fluorescence to judge the presence or absence of adenocarcinoma cells and/or atypical squamous cells. The apparatus may further have a scattered light detector, and the analysis unit may further analyze the detected scattered light. The apparatus may further have a imaging means, and the analysis unit may further analyze the image.

In consideration of individual features of gland cells and squamous cells and by paying attention to a marker specific to each cancer cell, the method for screening cervical cancer according to the present invention involves examining the presence or absence of the reactivity of a marker to each antibody thereby enabling not only discrimination of cancer cells from normal cells but also discrimination of adenocarcinoma cells from squamous carcinoma cells and further judging their precancerous state, and is thus utilizable effectively in diagnosis requiring judgment of the type of cervical cancer. Further, flow cytometry can be applied, whereby cervical cancer can be screened more efficiently.

The method for screening cervical cancer according to the present invention is capable of screening the presence or absence of cervical cancer and/or squamous cell carcinoma with high accuracy and high efficiency, and is thus useful in cellular diagnosis for cervical cancer wherein a large number of samples should be treated in medical examinations.

### BRIEF DESCRIPTION OF THE DRAWINGS

Fig. 1 is a flow chart for showing one embodiment of the screening method of the present invention.
Fig. 2 is a block diagram showing a structure in one example of a flow cytometer to which the screening method of the present invention can be applied.
Fig. 3 is an illustration for showing a fluorescence parameter distribution serving as the principle of the screening method of the present invention.
Fig. 4 is a flow chart for showing another embodiment of the screening method of the present invention.
Fig. 5 is a flow chart for showing an embodiment of the screening method of the present invention where a cell image is photographed.
Fig. 6 is a block diagram for showing one example of the constitution of a flow cytometer provided with a imaging means.
Fig. 7 is a flow chart for showing an embodiment of the screening method of the present invention where nuclei are stained.
Fig. 8 is a fluorescence profile (a), a side scattered light profile (b) and a forward scattered light profile (c) to show an application where the scattered light parameter is measured in the screening method of the present invention.
Fig. 9 is a side scattered light profile (a) and fluorescence profile (b) to show an application where nuclei were stained in the screening method of the present invention.
Fig. 10 is a fluorescence microphotograph (x400) showing the result of reaction of a first labeled antibody-containing reagent with HeLa cells, C33A cells or clinical samples 1 and 2.
Fig. 11 is a histogram obtained by applying HeLa cells and the first labeled antibody-containing reagent to a flow cytometer.
Fig. 12 is a histogram obtained by applying C33A cells and the first labeled antibody-containing reagent to a flow cytometer.
Fig. 13 is a histogram obtained by applying normal gland cells and the first labeled antibody-containing reagent to a flow cytometer.
Fig. 14 is a histogram obtained by applying normal squamous cells and the first labeled antibody-containing reagent to a flow cytometer.
Fig. 15 is a fluorescence microphotograph (x400) showing the result of reaction of a second labeled antibody-containing reagent with HeLa cells, C33A cells or clinical samples 1 and 2.
Fig. 16 is a histogram obtained by applying HeLa cells and the second labeled antibody-containing reagent to a flow cytometer.
Fig. 17 is a histogram obtained by applying C33A cells and the second labeled antibody-containing reagent to a flow cytometer.
Fig. 18 is a histogram obtained by applying normal gland cells and the second labeled antibody-containing reagent to a flow cytometer.
Fig. 19 is a histogram obtained by applying normal squamous cells and the second labeled antibody-containing reagent to a flow cytometer.
Fig. 20 is a fluorescence microphotograph (x400) showing the result of reaction of a third labeled antibody-containing reagent with C33A cells o normal gland cells.
Fig. 21 is a histogram obtained by applying (a) normal gland cells or (b) C33A cells and the third labeled antibody-containing reagent to a flow cytometer.
Fig. 22 is a synthetic profile consisting of a nucleus staining fluorescence profile and a side scattered light profile obtained in the embodiment of Example 2.
Fig. 23 is a scatter diagram showing the distributed state of N/C obtained in the embodiment of Example 2.
Fig. 24 is a two-dimensional scatter diagram obtained by measuring the fluorescence intensity and forward scattered light pulse width derived from the first fluorescence label in a model sample of cervical adenocarcinoma cells in Example 3.
Fig. 25 is a two-dimensional scatter diagram obtained by measuring the fluorescence intensity and forward scattered light pulse width derived from the second fluorescence label in a model sample of cervical adenocarcinoma cells in Example 3.
Fig. 26 is a two-dimensional scatter diagram obtained by measuring the fluorescence intensity and forward scattered light pulse width derived from the third fluorescence label in a model sample of cervical adenocarcinoma cells in Example 3.
Fig. 27 is a two-dimensional scatter diagram obtained by measuring the fluorescence intensity and forward scattered light pulse width derived from the first fluorescence label in a model sample of cervical squamous carcinoma cells in Example 3.
Fig. 28 is a two-dimensional scatter diagram obtained by measuring the fluorescence intensity and forward scattered light pulse width derived from the second fluorescence label in a model sample of cervical squamous carcinoma cells in Example 3.
Fig. 29 is a two-dimensional scatter diagram obtained by measuring the fluorescence intensity and forward scattered light pulse width derived from the third fluorescence label in a model sample of cervical squamous carcinoma cells in Example 3.
Fig. 30 is a two-dimensional scatter diagram obtained by measuring the fluorescence intensity and forward scattered light pulse width derived from the first fluorescence label in a model sample of cervical normal squamous cells in Example 3.
Fig. 31 is a two-dimensional scatter diagram obtained by measuring the fluorescence intensity and forward scattered light pulse width derived from the second fluorescence label in a model sample of cervical normal squamous cells in Example 3.
Fig. 32 is a two-dimensional scatter diagram obtained by measuring the fluorescence intensity and forward scattered light pulse width derived from the third fluorescence label in a model sample of cervical normal squamous cells in Example 3.

### DETAILED DESCRIPTION OF THE PREFERRED EMBODIMENT

The method for screening cervical cancer according to a first aspect of the present invention is a method comprising the steps of preparing a measurement sample by reacting cervical cells with a first labeled antibody reacting with gland cells, a second labeled antibody reacting with adenocarcinoma cells and a third labeled antibody reacting with atypical cervical squamous cells, the antibodies being labeled with mutually distinguishable labels respectively, detecting the labels derived from the respective labeled antibodies bound to the cervical cells, and judging the presence or absence of adenocarcinoma cells and/or atypical squamous cells, on the basis of the detected labels. At least one of the labels is preferably a fluorescence label.

In the screening method described above, it is preferable that the judging step comprises the steps of judging whether the reaction of the cells in the measurement sample with the first labeled antibody is positive or negative on the basis of the detected first label of the first labeled antibody, then judging whether the reaction of the cells judged to be positive to the first labeled antibody with the second labeled antibody is positive or negative on the basis of the detected second label of the second labeled antibody, and judging whether the reaction of the cells judged to be negative to the first labeled antibody with the third labeled antibody is positive or negative on the basis of the detected third label of the third labeled antibody.

The method for screening cervical cancer according to a second aspect of the present invention is a method comprising the steps of preparing a measurement sample by reacting cervical cells with a first fluorescence-labeled antibody reacting with gland cells, a second fluorescence-labeled antibody reacting with adenocarcinoma cells and a third fluorescence-labeled antibody reacting with atypical cervical squamous cells, the antibodies being labeled with mutually distinguishable fluorescences respectively, irradiating the measurement sample with an exciting light, detecting the fluorescence emitted by the measurement sample, and judging the presence or absence of adenocarcinoma cells and/or atypical squamous cells, on the basis of the detected fluorescence.

In the methods of screening cervical cancer according to the first and second aspects of the present invention, the antibody in the first labeled antibody is preferably at least one selected from the group consisting of MUC1 antibody, cytokeratin 7 antibody, and cytokeratin 18 antibody, the antibody in the second labeled antibody is preferably at least one selected from the group consisting of cytokeratin 8 antibody and HIK1083 antibody, and the antibody in the third labeled antibody is preferably at least one selected from the group consisting of NMP179 antibody, p16^{INK4A} antibody, Ki-67 antibody, p53 antibody, p21 antibody, EMA antibody, CEA antibody and MIB-1 antibody.

In the method for screening cervical cancer according to the second aspect of the present invention, the judgment may be conducted by judging whether the reaction of the cells in the measurement sample with the first fluorescence-labeled antibody is positive or negative by measuring the intensity of the fluorescence derived from the first fluorescence label emitted by the cells, then judging whether the reaction of the cells judged to be positive to the first fluorescence-labeled antibody with the second fluorescence-labeled antibody is positive or negative by measuring the intensity of the fluorescence derived from the second fluorescence label emitted by the cells, and judging whether the reaction of the cells judged to be negative to the first fluorescence-labeled antibody with the third fluorescence-labeled antibody is positive or negative by measuring the intensity of the fluorescence derived from the third fluorescence label emitted by the cells (first judgment method), or alternatively the method for screening cervical cancer may comprise the steps of allowing the measurement sample to flow through a flow cell of a flow cytometer, irradiating the cells in the measurement sample with an exciting light, measuring the fluorescence emitted by the cells for the fluorescence parameter derived from the first fluorescence label, the fluorescence parameter derived from the second fluorescence label, and the fluorescence parameter derived from the third fluorescence label, and the judging step includes step of judging the presence or absence of adenocarcinoma cells and/or atypical squamous cells on the basis of the respective fluorescence parameter values (second judgment method).

In the second judgment method, the fluorescence parameter is selected preferably from the group consisting of fluorescence intensity, fluorescence pulse width and fluorescence pulse area. Further, the scattered light parameter on the cells in the measurement sample may be measured to judge the presence or absence of adenocarcinoma cells and/or atypical squamous cells. In this case, the scattered light parameter is selected preferably from the group consisting of forward scattered light intensity, forward scattered light pulse width and side scattered light pulse width.

When the second judgment method is used, a two-dimensional scatter diagram having two axes wherein one kind of fluorescence parameter selected from the group consisting of the fluorescence parameter derived from the first fluorescence label, the fluorescence parameter derived from the second fluorescence label and the fluorescence parameter derived from the third fluorescence label is on one axis and the scattered light parameter is on the other axis may be prepared to judge the presence or absence of adenocarcinoma cells and/or atypical squamous cells.

Alternatively, when the second judgment method is used, cervical cells in the measurement sample are previously stained with a fluorescent dye capable of staining nuclei, and a fluorescence profile derived from staining of nuclei of the cervical cells, an emission profile not derived from staining of nuclei but derived from emission from the cervical cells, a forward scattered light profile and a side scattered light profile may be compared with one another to judge the presence or absence of adenocarcinoma cells and/or atypical squamous cells.

When the second judgment is used, the flow cytometer has a imaging means, and a cell image may be given by imaging the cells judged to be positive in reaction to the third fluorescence-labeled antibody.

In the method for screening cervical cancer according to the present invention, the cervical cells may be a cell population dispersed in single cells, or when the second judgment method is not used, the cervical cells may be cells on a smear sample.

The diagnostic reagent for cervical cancer according to the present invention comprises a first fluorescence-labeled antibody reacting with gland cells, a second fluorescence-labeled antibody reacting with adenocarcinoma cells and a third fluorescence-labeled antibody reacting with atypical cervical squamous cells, the antibodies being labeled with mutually distinguishable fluorescences respectively. It is preferable that the antibody in the first labeled antibody is at least one selected from the group consisting of MUC1 antibody, cytokeratin 7 antibody, and cytokeratin 18 antibody, the antibody in the second labeled antibody is at least one selected from the group consisting of cytokeratin 8 antibody and HIK1083 antibody, and the antibody in the third labeled antibody is at least one selected from the group consisting of NMP 179 antibody, p16^{INK4A} antibody, Ki-67 antibody, p53 antibody, p21 antibody, EMA antibody, CEA antibody and MIB-1 antibody.

The method of automatically diagnosing cervical cancer according to the present invention is a method wherein in the screening methods of the first and second aspects of the present invention, the presence or absence of adenocarcinoma cells and/or atypical squamous cells is automatically judged, and when the ratio of cells judged to be adenocarcinoma cells and/or atypical squamous cells to the cell population contained in the measurement sample is higher than a predetermined value, the cervical cancer is judged to be adenocarcinoma or squamous cell carcinoma.

The method for screening cervical cancer according to a third aspect of the present invention is a method comprising the steps of preparing a measurement sample by reacting cervical cells with an anti-gland cell labeled antibody reacting with gland cells and an anti-atypical squamous cell antibody reacting with atypical cervical squamous cells, the antibodies being labeled with mutually distinguishable labels respectively, detecting labels derived respectively from the respective labeled antibodies bound to the cervical cells, and judging the presence or absence of atypical squamous cells, on the basis of the detected labels. The antibody in the labeled antibody reacting with gland cells is preferably at least one selected from the group consisting of MUC1 antibody, cytokeratin 7 antibody, and cytokeratin 18 antibody, and the antibody in the labeled antibody reacting with atypical cervical squamous cells is preferably at least one selected from the group consisting of NMP179 antibody, p16^{INK4A} antibody, Ki-67 antibody, p53 antibody, p21 antibody, EMA antibody, CEA antibody and MIB-1 antibody.

Hereinafter, the present invention is described in more detail.

### [Diagnostic reagent for cervical cancer]

First, the diagnostic reagent for cervical cancer used in the method for screening cervical cancer according to the present invention is described in detail.

The diagnostic reagent for cervical cancer according to the present invention comprises a first antibody reacting with gland cells, a second antibody reacting with adenocarcinoma cells and a third antibody reacting with atypical cervical squamous cells, the antibodies being labeled with mutually distinguishable labels respectively.

As the first antibody reacting with gland cells, use is made of at least one member selected from the group consisting of MUC1 antibody, cytokeratin 7 antibody and cytokeratin 18 antibody.

The MUC 1 antibody is an antibody reacting with mucus in the surface of a cell. The gland cells produce mucus, while squamous cells do not produce mucus, so that due to the presence or absence of reaction with MUC1 antibody, gland cells containing mucus in the cells and squamous cells not containing mucus in the cells can be distinguished from each other.

Cytokeratin 7 antibody and cytokeratin 18 antibody are antibodies reacting specifically with cytokeratin 7 and cytokeratin 18 respectively. Cytokeratins belong to a protein group forming a filament of intermediate diameter as one of fibrous structures in cytoplasm. Cytokeratins 7 and 18 occur on various epithelial cells (including gland cells) but in a small amount in squamous cells. Cytokeratins 7 and 18 occur in tumor cells whether benign or malignant, but in a very small amount in squamous cell carcinoma. Accordingly, cytokeratins 7 and 18 antibodies are useful in discriminating gland cells.

As the second antibody reacting with adenocarcinoma cells, use is made of at least one selected from the group consisting of cytokeratin 8 antibody and HIK1083 antibody.

Cytokeratin 8 antibody is an antibody reacting specifically with cytokeratin 8. Cytokeratin 8 is contained not only in adenocarcinoma cells but also in squamous carcinoma cells, normal gland cells and squamous carcinoma cells, wherein the content of cytokeratin 8 is highest in adenocarcinoma cells. Accordingly, cytokeratin 8 antibody can bind predominantly to adenocarcinoma cells among normal cells, squamous carcinoma cells and adenocarcinoma cells, to detect adenocarcinoma cells.

HIK1083 antibody is an antibody reacting with mucin produced and secreted by gland mucous cells in the stomach, and immune staining with this antibody is known to be positive to cervical adenocarcinoma, particularly malignant adenoma (Ishii K. *et al*., Cancer 1999;87:245-253,).

As the third antibody, it is preferable to employ at least one selected from the group consisting of NMP179 antibody, p16^{INK4A} antibody, Ki-67 antibody, p53 antibody, p21 antibody, EMA antibody, CEA antibody and MIB-1 antibody.

NMP 179 antibody is a monoclonal antibody recognizing a nuclear matrix antigen related to cervical cancer, is a useful marker for early finding of squamous intraepithelial lesions in the precancerous state of cervical regions, and is useful for finding high-degree squamous intraepithelial lesions (HSIL) and light squamous intraepithelial lesions (LSIL).

Expression of p16^{INK4A} protein is known to be inducible by E7 protein of human papilloma virus (HPV), and cervical cancer is often caused by infection with HPV, so there are a large number of studies on p16^{INK4A} antibody as a cellular diagnostic marker of cervical cancer (Bibbo M*. et al.,* Acta Cytol. 46:25-29, 2002, Klaes R. *et al*., Int. J. Cancer, 92:276-284, 2001).

Because the expression of Ki-67 protein is promoted in growing cells, Ki-67 antibody is used for monitoring the growth of tumor. In studies on cervical cell analysis, there are a plurality of reports using Ki-67 antibody. MIB-1 antibody is one subtype of Ki-67 antibody (Pirog EC. *et al.,* 26(1):70-75, 2002; Pesnick M. *et al.,* Hum. Pathol., 27(3):234-239, 1996).

p53 protein has a tumor suppressing action but when its structure is changed, the cancer suppressing action is lost. Accordingly, mutant p53 antibody is used widely as a cancer marker. Studies using mutant p53 antibody have also been made on cellular analysis of cervical cancer (Maeda MY. *et al.,* Pathologica, 93:189-195, 2001, Kerstens HM. *et al.,* J. Histochem. Cytochem. 48:709-718, 2000).

p21 and p27 proteins, similar to p53 and p16^{INK4A}, are cell cycle-related proteins, and their promoted expression in cancer cells is recognized in various cancers. Accordingly, there are many studies and reports using p21 antibody as a marker of cancers including cervical cancer (van de Putte G. *et al.,* Gynecol. Oncol. 89:140-147, 2003, Graflund M. *et al.,* Int. J Gynecol. Cancer, 12:290-298, 2002).

EMA is a tumor cell-specific membrane protein, and thus there are a large number of studies and reports using it as a marker for cellular diagnosis of cervical adenocarcinoma cells (Sincock AM. *et al.,* J. Clin. Pathol. 36:535-538, 1983, Moncrieff D. *et al.,* Acta. Cytol. 28(4):407-410, 1984).

CEA protein is expressed in a large amount in cancer cells, and thus there are a large number of studies and reports using CEA antibody as a marker for cellular diagnosis of cervical adenocarcinoma cells (Bamford PN. *et al.,* Obstet. Gynecol. 61 (5):603-608, 1983).

Any of the antibodies used in the present invention is an antibody having a binding site to a marker as the subject of reaction, and may be a commercial antibody without particular limit to the structure of Fc fragment and a constant region of Fab. For example, anti-MUC1 antibody from Exapha Biologicals Inc. can be used as MUC1 antibody, and "Anti-Cytokeratin 8, 35H11 Mouse Monoclonal Antibody" commercially available from DAKO can be used as cytokeratin 8 antibody. NMP179 antibody is a product of Matritech.

p16^{INK4A} antibody, Ki-67 antibody, p53 antibody, p21 antibody, p27 antibody and CEA antibody are available as commercial products from DAKO. MIB-1 antibody is available from Immunotech.

In the diagnostic reagent of the present invention, the first, second and third antibodies preferably contain physiological saline, a phosphate or Tris buffer, a surfactant etc. in their solvent; specifically, a mixture of phosphate buffered physiological saline (PBS) and a Tween or Triton surfactant is used as the solvent.

### [With respect to the label of the antibody]

The first, second and third antibodies contained in the diagnostic reagent are labeled respectively with mutually distinguishable labels. Labeling may be conducted by binding a labeled compound directly to each antibody (direct method), or using the above antibodies as unlabeled primary antibodies, labeled secondary antibodies specific respectively to the primary antibodies may be bound to the primary antibodies thereby indirectly labeling the antibodies (indirect method).

When the respective antibodies are derived from animals of the same species, the antibodies are preferably labeled by the direct method in order to avoid erroneous judgment caused by cross reaction. When use is made of antibodies derived from animals of different species, which hardly undergo cross reaction, then the antibodies may be labeled by the indirect method using secondary antibodies.

The labeled compound that can be used in the present invention includes, but is not limited to, conventionally known labeled compounds, for example, fluorescent substances such as cyanine dyes such as Cy3 (registered trademark of Amersham Life Science), fluorescein isothiacyanate (FITC), allophycocyanin, rhodamine, quantum dot etc.; light scattering substances such as gold particles etc.; photo-absorptive substances such as ferrite; radioactive substances such as ¹²⁵I etc.; and enzymes such as peroxidase, alkali phosphatase etc. Among these, the three antibodies (first, second and third antibodies) used in the present invention are preferably labeled with fluorescent dyes different in fluorescence wavelengths because the labels of the antibodies can be easily discriminated from one another. The discrimination of the antibodies by dyes emitting 3 colors, for example by labeling the first antibody in red (for example PE-Cy5), the second antibody in orange (for example PI, APC, R-PE) and the third antibody in green (for example Alexa488, FITC) is preferably used.

### [Preparation of the measurement sample]

The measurement sample subjected to the screening method of the present invention is prepared in the following manner.

First, the reagent for diagnosing cervical cancer comprising the first labeled antibody, the second labeled antibody and the third labeled antibody according to the present invention is added to cervical cells to be screened, and then reacted for a predetermined time to prepare the measurement sample.

The cervical cells may be a cell population dispersed in single cells or may be cells on a smear sample. For application to flow cytometry excellent in processing speed, a cell population dispersed in single cells is preferably used.

As the cervical cells used in preparing the measurement sample, a group of cervical cells collected by scrubbing the cervical surface with a cotton swab or a scrubber may be used as such, or the cells freed from mucus and then reacted with the reagent for diagnosing cervical cancer according to the present invention may be used. The group of cervical cells collected by scrubbing the cervical surface with a cotton swab or a scrubber contains gland cells secreting mucus, and by the mucus, the reaction with the antibodies contained in the reagent may not sufficiently proceed, which can be prevented by previously removing the mucus. Particularly for application to flow cytometry, cellular mass in a state aggregated with the mucus cannot be applied to flow cytometry, and thus the mucus is preferably removed.

The method of removing the mucus is not particularly limited, but treatment with a cysteine compound proposed by the present inventors is preferably used. As the cysteine compound, methyl cysteine, acetyl cysteine, L-cysteine etc. can be used.

When flow cytometry is used, the cells after removal of mucus are preferably dispersed into individual cells. This is because given only the mucus removal treatment, the cells may be hardly dispersed into completely individual cells. When the cells are imaged as described later in the screening method of the present invention, dispersing treatment should be conducted without destroying the form of cells. The method of dispersing the cells without destroying their form preferably involves stabilizing the cells with an aldehyde compound and then treating the cells with a proteolytic enzyme, as proposed in e.g. Japanese Patent Application 2003-359336 by the present inventors. This is because when the cells are treated with a proteolytic enzyme without stabilization treatment, the cells can be destroyed. As the aldehyde compound, paraformaldehyde, formaldehyde, glutaraldehyde or a mixture thereof can be used.

The proteolytic enzyme used in dispersing the cells includes trypsin, pronase, pepsin, elastase, collagenase etc., among which collagenase is preferably used. An enzyme having low digestion power is preferably used because the time necessary for dispersion can be determined in a relatively broad range and thus a difference in suitable digestion time of individual samples cannot be problematic.

The conditions for reaction of cervical cancer cells and the diagnostic reagent are not particularly limited, but the reaction is conducted preferably at room temperature for about 1 to 30 minutes, and shaking or the like is preferably conducted to improve the reaction efficiency.

Prior to the reaction with the diagnostic reagent, non-specific protein in the sample is preferably blocked. The blocking reagent is preferably a dilution of bovine albumin, control serum or casein with physiological saline or the like.

When the cells are reacted with secondary antibodies after the blocking reaction and the subsequent reaction with the diagnostic reagent, the measurement sample is preferably washed after the reaction with the secondary antibodies.

### [Principle of the method for screening cervical cancer]

The screening method of the present invention is a method of judging the presence or absence of adenocarcinoma cells and/or atypical squamous cells by using the measurement sample prepared above. The principle of the method is based on the presence or absence of the reaction with the first, second and third antibodies respectively. That is, the measurement sample is positive (+) when the sample reacts with the objective antibody, while the sample is negative (-) when the sample does not react with it, and whether the sample is positive and/or negative to the first, second and third antibodies is judged, and from the combination of the results, the sample is classified as shown in Table 1. The cell positive to all of the first, second and third antibodies is classified into group I (adenocarcinoma cells); the cell positive to the first and third antibodies but negative to the second antibody is classified into group II (squamous metaplasia cells, gland cells); the cell negative to the first and second antibodies but positive to the third antibody is classified into group III (atypical calls, squamous carcinoma cells, squamous cells of basal cell layer); and the cell negative to all of the first, second and third antibodies is classified into group IV (squamous cells of the epithelial layer and intermediate layer).

**Table 1**

| | Group I | Group II | Group III | Group IV |
|---|---|---|---|---|
| First antibody | + | + | - | - |
| Second antibody | + | - | - | - |
| Third antibody | + | + | + | - |
| Cell | adenocarcinoma cell | metaplasia cell, gland cell | squamous cell (basal layer), atypical cell, squamous carcinoma cell | normal squamous cell (epithelial layer and intermediate layer) |

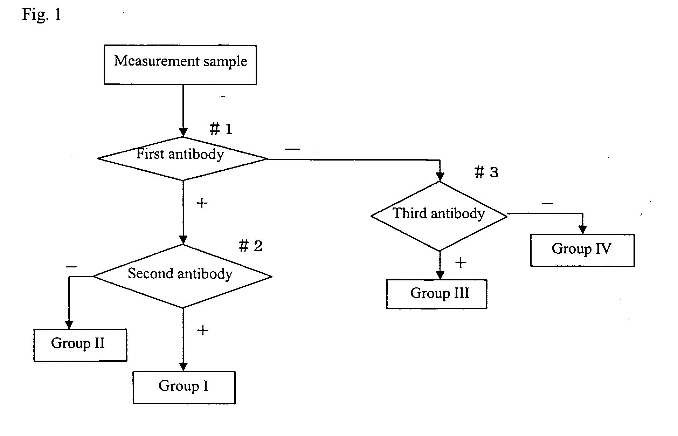

In the screening method of the present invention, the measurement sample prepared by reacting the first, second and third labeled antibodies with cervical cells may be examined for the presence of absence of the reaction with each antibody, based on the label of each antibody, to judge which of the classes shown in Table 1 the sample cell belongs to, or the cell can be judged based on the flow chart shown in Fig. 1.

That is, the presence or absence of the reaction with the first antibody is judged (step #1), and when the cell is positive, the presence or absence of its reactivity with the second antibody is judged (step #2), and when the cell is negative to the first antibody, the presence or absence of its reactivity with the third antibody is judged (step #3). According to the procedure in this order, whether a cell classified into any of groups I to IV the cell is contained or not may be judged.

The presence or absence of the reaction is judged based on the label bound to each antibody. For example, when a fluorescence label is used, the measurement sample can be irradiated with an exciting light to determine which of the fluorescence labels the fluorescence emitted from the measurement sample is based on. For example, whether the cell is positive or negative to each antibody may be judged by observing the emitted fluorescence color under a microscope, or which of the labels the fluorescence is based on may be examined by using a frequency spectrum of the emitted fluorescence. Which of the labeled antibodies the emitted fluorescence is derived from may be examined by measuring the intensity of the fluorescence passing through a specific band path filter.

### [Use of flow cytometry]

When a cell population dispersed in single cells is used as the cervical cells subjected to measurement, flow cytometry can be utilized in screening. When a flow cytometer is used in screening, a fluorescent substance is preferably used in labeling each antibody.

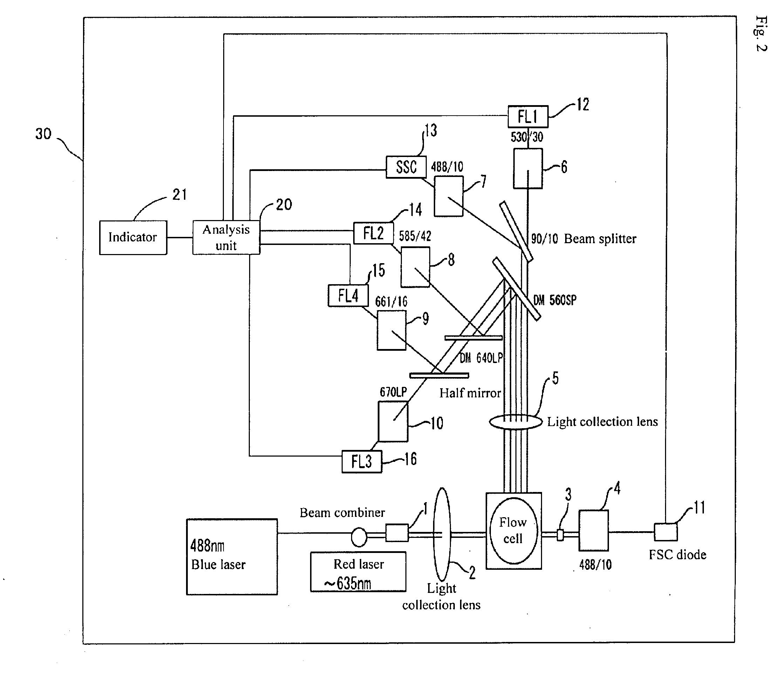

Which of the fluorescence-labeled antibodies the cells passing a flow cell react with can be known for example by using a flow cytometer having the constitution as shown in Fig. 2.

In apparatus 30 shown in Fig. 2, a blue laser (Ar ion laser) having an oscillation wavelength of 488 nm and a red laser (semiconductor layer) having an oscillation wavelength of 635 nm are combined in a beam combiner 1 to form an exciting light. The formed exciting light passes through a light collection lens 2 and regulated so as to have a flat beam profile having a minor axis of about 10 µm and a major axis of about 100 µm, with which the flow cell is to be irradiated. The exciting light emitted by the light collection lens 2 passes through the flow cell, to form an image on a beam stopper 3 by which the primary light is stopped. From the light scattered by the cells, the fluorescence emitted by the cells is cut by filter 4 (interference filter with a central wavelength of 488 nm and a pass wavelength of 10 nm), and the scattered light at a solid angle of a few degrees viewed from forward scattered light detector 11 (FSC diode) is collected and detected in forward scattered light detector 11.

On one hand, the fluorescence emitted from the cells is collected by a light collection lens 5 having a high number of aperture (NA) arranged in the side of the flow cell. The light emitted by the light collection lens 5 is passed through a dichroic mirror (DM560SP) inherently passing a light of shorter wavelength than 560 nm. The light passing through this mirror is resolved by a beam splitter having a resolution ratio of 90 : 10, and a light having a resolution ratio of 10% passes through an interference filter 6 having a central wavelength of 530 nm and a pass wavelength of 30 nm and enters FL1 detector 12 (photomultiplier tube (PMT)) where green fluorescence is detected. On the other hand, a light having a resolution ratio of 90% passes through an interference filter 7 having a central wavelength of 488 nm and a pass wavelength of 10 nm and enters SSC detector 13 (photomultiplier tube (PMT)) where side scattered light is detected. Then, the light reflected by a dichroic mirror (DM560SP) is passed through a dichroic mirror (DM640LP) inherently passing a light having a wavelength longer than 640 nm. Fluorescence having a wavelength between 560 nm to 640 nm is reflected by this mirror, passes through an interference filter 8 having a central wavelength of 585 nm and a pass wavelength of 42 nm and enters FL2 detector 14 (photomultiplier tube (PMT)) where orange light is detected. Fluorescence having a wavelength longer than 640 nm is resolved at 50 : 50 by a half mirror, and one divided fluorescence passes through an interference filter 9 having a central wavelength of 661 nm and a pass wavelength of 16 nm and enters FL4 detector 15 (photomultiplier tube (PMT)) where red fluorescence is detected. The other divided fluorescence passes through an interference filter 10 having a wavelength of 670 nm or higher and enters FL3 detector 16 (photomultiplier tube (PMT)) where ultra-red fluorescence is detected. In the present invention, at least 3 of 4 fluorescences described above may be used.

Each signal of the forward scattered light, side scattered light, green fluorescence, orange fluorescence, red fluorescence or ultra-red fluorescence is sent to an analysis unit 20, and each output signal is subjected to desired analysis described below to display results in indicator 21.

The measurement sample prepared using the diagnostic reagent having each antibody labeled with fluorescence is passed through the flow cell of the flow cytometer capable of multicolor analysis, and each cell passing through the flow cell is irradiated with an exciting light (for example, a blue and/or red laser from a laser light source in the apparatus in Fig. 2) having a flat intensity profile of several hundreds µm×a few µm, and the fluorescence emitted from the cells passing through the detecting zone is measured for the fluorescence parameter derived from the first fluorescence label, the fluorescence parameter derived from the second fluorescence label and the fluorescence parameter derived from the third fluorescence label respectively. In the apparatus in Fig. 2, the fluorescence derived from each labeled antibody can be detected in any of FL1 to FL4, depending on the type of fluorescence, and the desired fluorescence parameter value can be calculated.

The fluorescence parameter used is preferably one kind of parameter selected from the group consisting of fluorescence intensity, fluorescence pulse width and fluorescence pulse area, or a combination thereof. The fluorescence intensity is the maximum value in the fluorescence profile of cells. The fluorescence pulse area is an integrated value of the fluorescence profile. The fluorescence pulse width is a time in which the fluorescence pulse exceeds a predetermined threshold value.

According to the flow cytometer, the fluorescence of the individual cells passing through the flow cell is detected, and a fluorescence profile having time on the abscissa and fluorescence parameter such as fluorescence intensity on the ordinate is prepared, whereby the presence or absence of the reactivity of the individual cells to the first, second and third antibodies can be judged. On the basis of this judgment result, which of groups I to IV in Table 1 the individual cells belong to can be judged according to the flow shown in Fig. 1. Further, a histogram of the cell group contained in the measurement sample, where the number of cells is counted for each fluorescence parameter value based on each labeled antibody, can be prepared to determine how much cells contained in adenocarcinoma cells (group I) and squamous carcinoma cells (group III) are present in the cell group in one measurement sample.

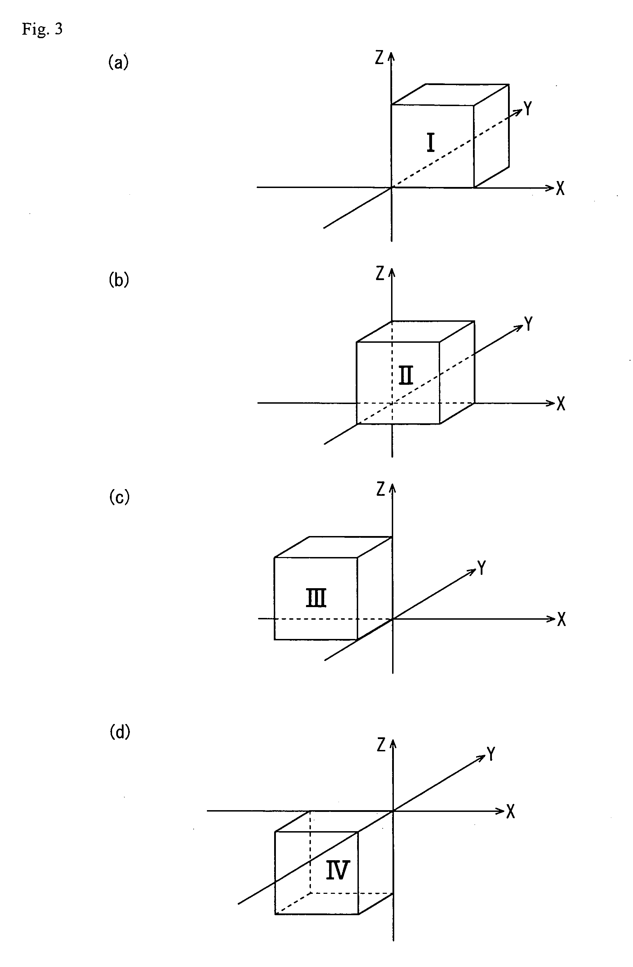

The individual fluorescence labels of the first, second and third labeled antibodies are thus successively judged. Further, in three-dimensional coordinates wherein for example, the fluorescence parameter based on the first fluorescence-labeled antibody is shown on the X-axis, the fluorescence parameter based on the second fluorescence-labeled antibody on the Y-axis, and the fluorescence parameter based on the third fluorescence-labeled antibody on the Z-axis, the fluorescence parameter values of the first, second and third fluorescence-labeled antibodies measured are plotted, and depending on the position of the three-dimensional space shown in Fig. 3(a) to (d), which of groups I to IV the cells belong to can be easily judged. In this case, the whole of the cell group contained in one measurement sample can be dot-plotted to judge where the density of plots is high in the three-dimensional space in Fig. 3(a) to (d) thereby analyzing the amount of cells in the sample contained in adenocarcinoma cells (group I) and/or squamous carcinoma cells (group III).

By application of flow cytometry as described above, whether individual cells are positive or negative to the first, second and third antibodies can be judged to determine whether adenocarcinoma cells and/or atypical squamous cells are contained in the measurement sample. By examining the distributed state of fluorescence parameter values of all cells contained in one measurement sample corresponding to an individual sample, the degree of occurrence of the cells belonging to groups I to IV can be known.

With respect to the analysis of measurement results of one measurement sample corresponding to one sample subjected to flow cytometry, it is possible to apply not only a frequency histogram showing the relationship between fluorescence parameter and the number of cells or plots on the three-dimensional coordinates in Fig. 3, but also various display methods and analysis methods by known programs, such as two-dimensional contour plotting wherein elements of the same frequency are bound to one another via a contour line, equivalent plotting using a Z-axis, and diamond display using a plurality of parameters. With respect to plotting, processing by gating and window may be conducted if necessary.

### [Screening method where scattered light parameter is measured]

In application of the screening method of the present invention to flow cytometry, not only the fluorescence based on the label but also the scattered light generated from the cells passing through the detecting zone in the flow cell is preferably measured. In the apparatus shown in Fig. 2, the forward scattered light can be detected by FSC diode and the side scattered light by SSC detector.

By measuring the fluorescence parameter and scattered light parameter of the cells passing through the flow cell, further detailed information on the cells can be obtained, and pseudo-positive reaction due to cell debris can be eliminated. In some cases, the cell debris is positive to the third antibody so that in judgment by only the fluorescence parameter, the cells can be classified in group III. When the number of plots in group III is increased due to cell debris in a prepared scatter diagram of the measurement sample (i.e. the cell population), the cells may be misjudged to be squamous carcinoma cells, and thus elimination of pseudo-positive reaction based on cell debris is important in the screening method of the present invention and in the method of automatically diagnosing cervical cancer according to the present invention.

As the scattered light parameter, at least one selected from the group consisting of forward scattered light intensity (FSCP), forward scattered light pulse width (FSCW) and side scattered light pulse width (SSCW), each reflecting the size of cell, can be used. As the parameter reflecting the complexity of intracellular structure, side scattered light pulse height (SSCP) can also be used.

The pulse width corresponds to the time in which a pulse appears (that is, the time in which one cell flows) when a profile of scattered light upon measurement during which a single cell passes through the detecting zone in the flow cell is prepared with pass time on the abscissa and scattered (forward scattered, side scattered) light intensity on the ordinate.

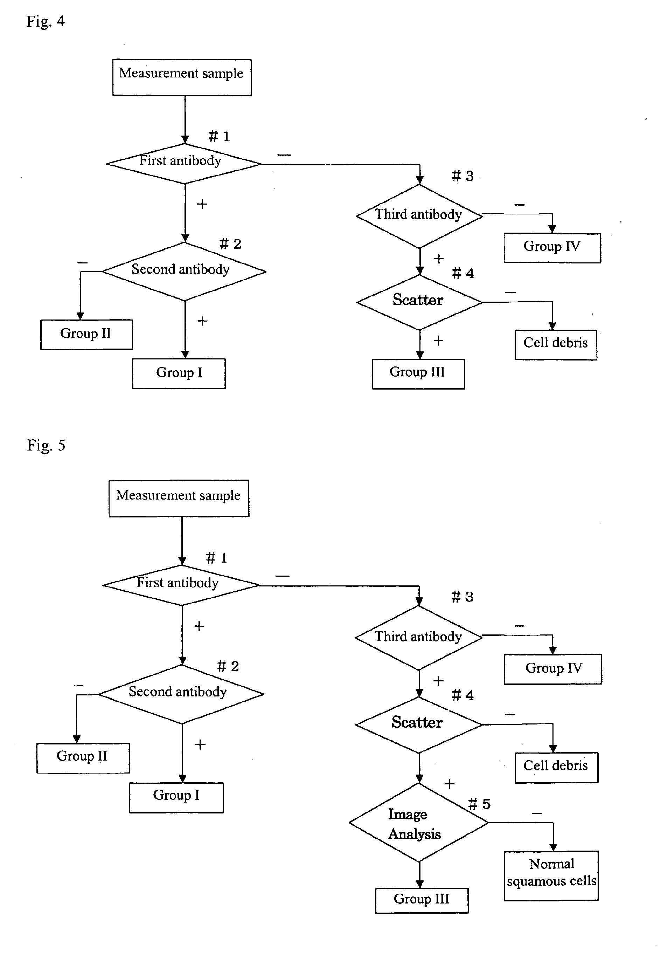

Fig. 4 shows one embodiment of the screening method of the present invention wherein the scattered light parameter reflecting the cell size and the fluorescence parameter are measured to eliminate cell debris contained in the measurement sample.

In the embodiment shown in Fig. 4, cells passing through the flow cell are measured for the fluorescence parameter and for the scattered light parameter reflecting the cell size to judge the presence or absence of the reaction with the first labeled antibody in step #1, and the cell judged to be positive to the first labeled antibody is used to judge the presence or absence of the reaction with the second labeled antibody (step #2). On one hand, the cell judged to be negative to the first labeled antibody is used to judge the presence or absence the reaction with the third labeled antibody (step #3), and the cell judged to be positive to the third labeled antibody is used to prepare a two-dimensional scatter diagram with the fluorescence parameter and scattered light parameters as the two axes in step #4, and from the position of plots, whether the cell is cell debris or not is judged. When it is judged that the fluorescence intensity to the third antibody is high, but the scattered light parameter value is remote from the cell size (as shown in "-" in Fig. 4), the cell is judged to be cell debris, and in another case (as shown in "+" in Fig. 4), the cell is judged to be a cell classified into group III.

As the scattered light parameter, the side light scattered light pulse height (SSCP) that is a parameter reflecting the complexity of intracellular structure is measured, and a two-dimensional scatter diagram having side light scattered light pulse height (SSCP) and fluorescence parameter can be prepared to know the frequency of appearance of atypical cell. That is, when the cancerous change of normal cells advances, the cells become small to make their intracellular structure complex, so that when the parameter (SSCP) on intracellular complexity is high relative to the parameter (SSCW) on cell size, the cells are considered as atypical cells. Accordingly, when the density of plots in a region indicative of atypical cells in the two-dimensional scatter diagram is high, the fluorescence parameter based on the third antibody is corrected positively, whereby the accuracy of diagnosis of cancer can be improved upon weighting in the method of automatically diagnosing cervical cancer as described later.

### [Screening method including a step of judgment of a cell image]

For application to a flow cytometer equipped with a imaging means, a cell image is produced if necessary, and the cells may be specifically judged from the state of the cells.

Fig. 5 shows one embodiment of the screening method using a flow cytometer equipped with a imaging means. According to this flow chart, when a cell positive to the third antibody are actually cell debris, the cell is eliminated in step #4, and when a cell is not cell debris, a cell image of the cell is prepared (step #5).

The cells judged to be positive to the third antibody are squamous carcinoma cells or atypical squamous cells in a precancerous state. When normal atypical squamous cells in the basal cell layer are contained, these squamous cells in the basal cell layer are also positive to the third labeled antibody. However, the normal squamous cells are different in form and nucleus/cytoplasm ratio from squamous carcinoma cells and atypical squamous cells, and thus can be distinguished by observing the form of the cells. A cell image of only the cells judged to be negative in step #1 and positive in step #3 is merely prepared, and thus the influence on processing speed is low.

According to the image, the presence or absence of the aggregation of the cells can be known. For example, when cells such as leucocytes irrelevant to squamous carcinoma cells but reacting with the third antibody are aggregated with normal squamous cells, the cells which should be classified into normal squamous cells (group IV) become cells positive to the third antibody, are thus classified into group III and erroneously judged to be squamous cell carcinoma. By forming an image in step #5 in this case, whether the cells are aggregated cells or not can be known, and whether the aggregated cells are leucocytes or not can be judged. When the cells passing through the flow cell are thus judged to be aggregated cells, the aggregated cells are exceeded from the subject to be analyzed. In this manner, the erroneous classification of normal squamous cells into group III because of their positive reaction to the third antibody can be prevented, thus reducing errors in analysis results of the cell group as one sample expressed in a histogram or the like.

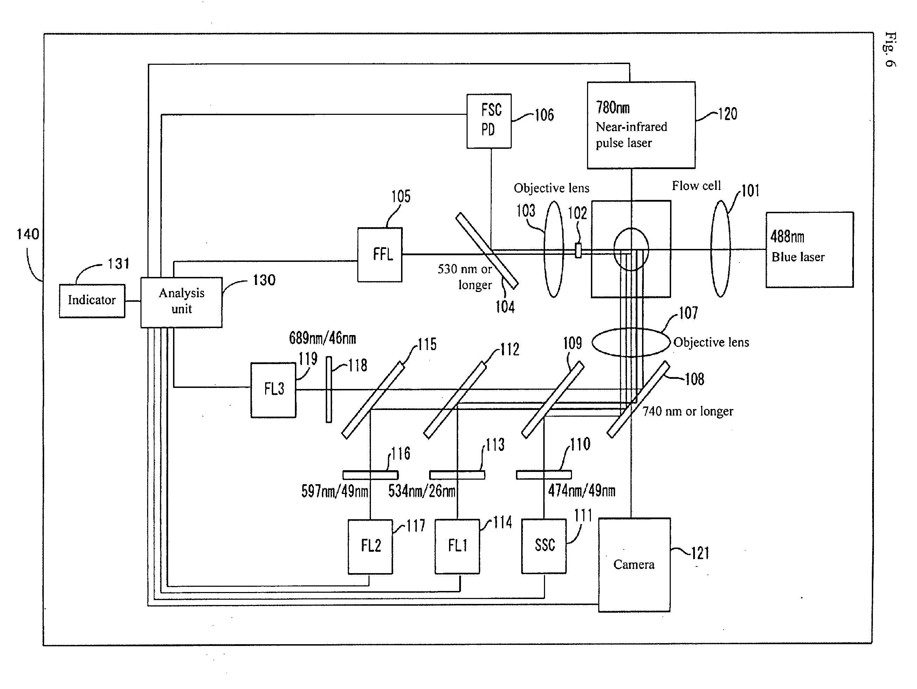

As the flow cytometer having a imaging means, an apparatus having the constitution shown in Fig. 6 can be used. In the apparatus 140, the fluorescence from the cells passing through the flow cell is detected by FL1 to FL3, and the forward scattered light is detected by FSC, and the side scattered light by SSC, and the cells are imaged by a camera.

In more detail, a blue laser (Ar ion laser) having an oscillation wavelength of 488 nm is passed through lens 101 and regulated so as to have a flat beam profile of a minor axis of about 10 µm and a major axis of about 100 µm, with which the flow cell is to be irradiated.

The exciting light emitted by the lens 101 passes through the flow cell, to form an image on a beam stopper 102 by which the primary light is stopped. The fluorescence/scattered light from the cells is collected by an objective lens 103, then passes through dichroic mirror 104 inherently passing a light having a wavelength of 530 nm or longer, and fluorescence having a solid angle of about 10° enters detector 105 (photomultiplier tube (PMT)) where forward fluorescence (FFL) is detected. A scattered light of a solid angle of about 10°, in fluorescence having a wavelength of 530 nm or shorter, enters detector 106 (photodiode: PD) where forward scattered light (FSC) is detected.

On one hand, the fluorescence/scattered light emitted by the cells is collected by an objective lens 107 having a high number of aperture (NA) arranged in the side of the flow cell. The light emitted by the objective lens 107 is passed through a dichroic mirror 108 inherently reflecting a light having a wavelength shorter than 740 nm.
The side fluorescence/scattered light reflected by mirror 108 is first passed through dichroic mirror 109 inherently reflecting a light having a wavelength of 500 nm or shorter, then passes through an interference filter 110 having a central wavelength of 474 nm and a pass wavelength of 49 nm and enters SSC detector 111 (photomultiplier tube (PMT)) where side scattered light is detected. The light passing through the dichroic mirror 109 enters dichroic mirror 112 inherently reflecting a light having a wavelength of 550 nm or shorter, passes through an interference filter 113 having a central wavelength of 534 nm and a pass wavelength of 26 nm and enters FL1 detector 114 (photomultiplier tube (PMT)) where green fluorescence is detected.

The light passing through the dichroic mirror 112 is resolved by dichroic mirror 115 into a light of 630 nm or shorter and a light of 630 nm or longer. One divided light passes through an interference filter 116 having a central wavelength of 597 nm and a pass wavelength of 49 nm and enters FL2 detector 117 (photomultiplier tube (PMT)) where orange fluorescence is detected, while the other divided light passes through an interference filter 118 having a central wavelength of 689 nm and a pass wavelength of 46 nm and enters FL3 detector 119 (photomultiplier tube (PMT)) where red fluorescence is detected. In the present invention, detection of the forward fluorescence (FFL) is not always necessary.

The captured forward scattered light (FSC), forward fluorescence (FFL), side scattered light (SSC), green fluorescence (FL1), orange fluorescence (FL2) and red fluorescence (FL3) are converted into A/D and input to an analysis unit where these are converted into signals in real time, and when these signals have a certain feature, a trigger signal is sent from the analysis unit, to emit a near-infrared pulse laser 120 having an oscillation wavelength of 780 nm. The pulse laser 120 acts as a transmitting light, and the light emitted by the flow cell passes through the first dichroic mirror 108 to form an image on camera 121, and the imaging data are sent to the analysis unit 130. A static image of the cells having arbitrary scattered light and fluorescence can thus be captured.

In the analysis unit 130, various kinds of analysis described above are conducted, and the results are shown in an indicator 131.

### [Screening method using the measurement sample subjected to nucleus staining]

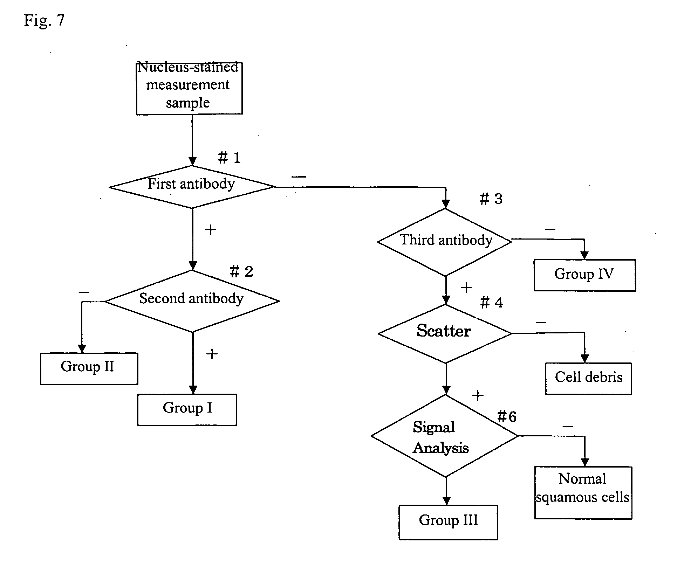

Fig. 7 shows one embodiment of the screening method wherein the measurement sample previously subjected to nucleus staining is applied to flow cytometry.

Staining of nuclei of all cells contained in the measurement sample is carried out using a fluorescent dye. As the fluorescent dye used in staining of nuclei, a fluorescent dye distinguishable from the fluorescence used in labeling of the first, second and third antibodies is used for 4-color analysis. For example, a flow cytometer having 4 kinds of FL1 to FL4 fluorescence detectors as shown in Fig. 2 can be used in measurement.

In the flow chart shown in Fig. 7, the third antibody-positive cells from which cell debris was removed can be examined by analyzing the parameter derived from nucleus staining and the scattered light parameter in step #6 thereby judging whether a cell aggregate is present or not or whether the cells passing through the flow cell are cancer cells or not, even if a time-consuming cell image is not prepared.

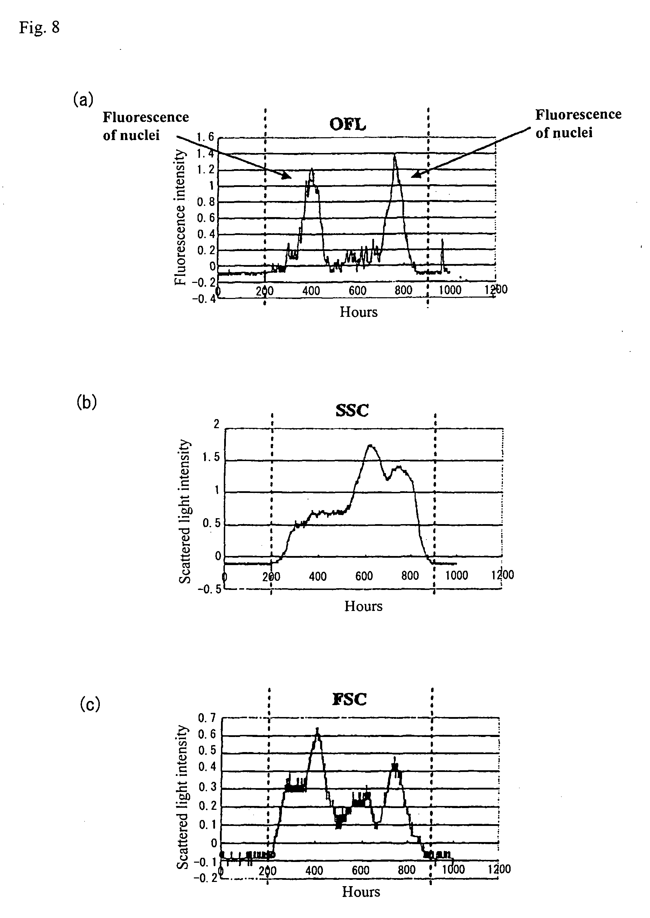

For example, a fluorescence profile and a scattered light profile where the time elapsed for the cell to pass through the detecting zone and the fluorescence intensity derived from staining of a nucleus of the cell are used as the two axes are prepared, and both the profiles are compared. As the fluorescence profile derived from nucleus staining where two cells are aggregated, an emission profile shown in e.g. Fig. 8(a) is obtained. On one hand, the side scattered light profile on the time for the cell to pass through the same detecting zone is for example as shown in Fig. 8(b), and the forward scattered light profile is for example as shown in Fig. 8(c). The pulse is not clearly separated in the scattered light profile in Fig. 8(b) or (c), so there is a high possibility that the sample is judged to be a single cell, while clearly separated two pulses appear in the nucleus staining fluorescence profile in Fig. 8(a), and it can thus be judged that tow nuclei, that is, an aggregate of two cells, pass per unit time. Accordingly, even if the cells are not imaged, judgment by the nucleus staining profile in addition to the scattered light profile enables detection of cell aggregates to eliminate them from the subject of analysis.

In step #6, a scattered light profile and a fluorescence profile of cells passing through the flow cell may be compared to judge whether the cells are single cells or aggregated cells, or the time for a single cell to pass through the flow cell and a fundamental fluorescence profile derived from nucleus staining of the cell are stored as a database file, and a nucleus fluorescence profile obtained by measurement is compared with data of the fundamental profile, and when there is a difference in pattern, the cells can be judged as cell aggregates.

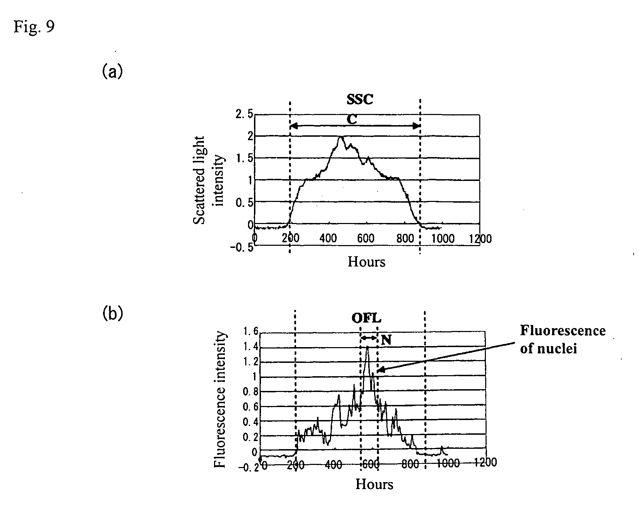

The size (N) of nucleus is determined from the fluorescence profile derived from nucleus staining, and the size (C) of cytoplasm is determined from the side scattered light pulse width (SSCW) of the cell, and the ratio of nucleus to cytoplasm (N/C) is determined. Specifically, in the SSC profile shown in Fig. 9(a), the width C of appearing pulse is cytoplasm size C, and e.g. the half width of the fluorescence profile in the nucleus fluorescence profile shown in Fig. 9(b) is nucleus size N. Cancer cells show a high N/C ratio, so that when the calculated N/C is higher than a predetermined value, the cells can be judged to be highly cancerous.

By analyzing the fluorescence profile derived from nucleus staining and the scattered light profile, normal squamous cells judged to be pseudo-positive can be prevented from being classified into group III by finding cell aggregates, and from positive cells (group III) to the third antibody, normal squamous cells (i.e. squamous cells in the basal cell layer) and squamous carcinoma cells can be discriminated, and thus the accuracy of screening of atypical squamous cells can be further improved.

### [Method of automatically diagnosing cervical cancer cells]

The method of automatically diagnosing cervical cancer according to the present invention is a method wherein according to the screening method of the present invention, the presence or absence of adenocarcinoma cells and/or atypical squamous cells is automatically judged, and when the ratio of cells judged to be adenocarcinoma cells and/or atypical squamous cells to the cell population contained in the measurement sample is higher than a predetermined value, the cervical cancer is judged to be adenocarcinoma or squamous cell carcinoma.

When flow cytometry is applied to the screening method, a histogram of the number of cells against the fluorescence parameter as the result of analysis of one sample, a scatter diagram wherein all cells contained in the measurement sample are plotted on three-dimensional coordinates of fluorescence parameter to each antibody as shown in Fig. 3, or a scatter diagram wherein all cells contained in the measurement sample are plotted on two-dimensional coordinates of fluorescence parameter and scattered light parameter of each antibody is prepared, and the cells can be diagnosed from the dispersed state of plots.

For example, when the density of plots in a region corresponding to groups I and III is higher than a predetermined ratio, the cells can be judged to be cervical cancer cells.

In judgment in automatic diagnosis, for example, side scattered light pulse height (SSCP) is measured as scattered light parameter, to prepare a two-dimensional scatter diagram with fluorescence parameter, and a certain area is divided and the frequency of appearance of atypical cells per area is examined, and when atypical cells appear frequently, the ratio that is a judgment standard may be corrected.

### [Method for screening atypical squamous cells]

The present invention can be applied to the screening method not only for judging the presence or absence of adenocarcinoma cells and/or atypical squamous cells but also for judging the presence or absence of atypical adenocarcinoma cells.

That is, this method is a method comprising the steps consisting of preparing a measurement sample by reacting cervical cells with an anti-gland cell labeled antibody reacting with gland cells and an anti-atypical squamous cell labeled antibody reacting with atypical cervical squamous cells, the antibodies being labeled with mutually distinguishable labels respectively, detecting labels derived respectively from the respective labeled antibodies bound to the cervical cells, and judging the presence or absence of atypical squamous cells, on the basis of the detected labels.

The antibody in the labeled antibody reacting with gland cells is preferably at least one selected from the group consisting of MUC1 antibody, cytokeratin 7 antibody, and cytokeratin 18 antibody, while the antibody in the labeled antibody reacting with atypical cervical squamous cells is preferably at least one selected from the group consisting of NMP179 antibody, p16^{INK4A} antibody, Ki-67 antibody, p53 antibody, p21 antibody, EMA antibody, CEA antibody and MIB-1 antibody. That is, the diagnostic reagent used in the screening method of judging the presence or absence of atypical squamous cells is a mixture of the first labeled antibody and/or the second labeled antibody and the third labeled antibody used in the above method for screening cervical cancer, and the same reagent as the diagnostic reagent used in the method for screening cervical cancer can also be used, or the reagent for diagnosing cervical cancer, from which the first or second labeled antibody was removed, can also be used.

When the sample is negative to the anti-gland cell-labeled antibody reacting with gland cells and positive to the anti-atypical squamous cell-labeled antibody reacting with atypical cervical squamous cells, it is judged that atypical squamous cells are present, and for discrimination from cell debris or for discrimination of atypical squamous cells in the normal basal cell layer contained in a very small amount in the sample from atypical squamous cells, a two-dimensional scatter diagram with fluorescence label parameter and scattered light parameter reflecting cell size may be formed as conducted in step #4 in Fig. 4, a cell image may be prepared as conducted in step #5 in the embodiment in Fig. 5, or the signal analysis conducted in the embodiment of Fig. 7 may be conducted.

### EXAMPLES

### [Preparation of the diagnosis reagent]

### (1) First labeled antibody-containing reagent

As the first labeled antibody, cytokeratin 7 antibody was used and labeled in the following manner to prepare a reagent containing the first labeled antibody.

Two µg/ml secondary labeled antibody (mouse IgG F(ab)₂ having Alexa488 (green fluorescent dye) bound thereto) was added to, and mixed with, the primary antibody solution diluted with PBS containing 1% goat serum such that the cytokeratin 7 antibody became 1 µg/ml, and the mixture was reacted for 5 minutes. Then, mouse IgG was added to a final concentration of 10 µg/ml thereto, and stirred for 5 minutes, to absorb an excess of the secondary antibody. As the diluent of the secondary antibody, PBS containing 1% goat serum was used.

### (2) Second labeled antibody-containing reagent

As the second labeled antibody, cytokeratin 8 antibody was used and labeled in the following manner to prepare a reagent containing the second labeled antibody.

Two µg/ml secondary labeled antibody (mouse IgG F(ab)₂ having R-PE (orange fluorescent dye) bound thereto) was added to, and mixed with, the primary antibody solution diluted with PBS containing 1% goat serum such that the cytokeratin 8 antibody became 1 µg/ml, and the mixture was reacted for 5 minutes. Then, mouse IgG was added to a final concentration of 100 µg/ml thereto, and stirred for 5 minutes, to absorb an excess of the secondary antibody. As the diluent of the secondary antibody, PPS containing 1% goat serum was used.

### (3) Third labeled antibody-containing reagent

As the third labeled antibody, NMP 179 antibody was used and labeled in the following manner to prepare a reagent containing the third labeled antibody.

Ten µg/ml secondary labeled antibody (mouse IgG F(ab)₂ having Alexa488 (green fluorescent dye) bound thereto) was added to, and mixed with, the primary antibody solution diluted with PBS containing 1% goat serum such that the NMP179 antibody became 7.4 µg/ml, and the mixture was reacted for 5 minutes. Then, mouse IgG was added to a final concentration of 250 µg/ml thereto, and stirred for 5 minutes, to absorb an excess of the secondary antibody. As the diluent of the secondary antibody, PBS containing 1% goat serum was used.

### [Used cells]

As a sample of adenocarcinoma cells, HeLa cells as cultured cells of cervical adenocarcinoma were used. As a sample of squamous carcinoma cells, C33A cells as cultured cells of cervical squamous cell carcinoma were used. As normal gland cells and normal squamous cells, normal clinical sample 1 abundant in gland cells (hereinafter referred to merely as "normal gland cells") and normal clinical sample 2 abundant in squamous cells (hereinafter referred to merely as "normal squamous cells").

### [Cell reactivity]

### Example 1

Four tubes were prepared, and HeLa cells, C33A cells and the clinical samples 1 and 2 were introduced at a density of about 1×10⁶ cells into the tubes respectively. The cells were fixed with PreservCyt solution from Cytyc Ltd. and then centrifuged at 10000 rpm for 1 minute to remove a supernatant. Then, 5% N-acetyl-L-cysteine was added thereto, and the cells were stirred and centrifuged at 10000 rpm for 1 minute to remove a supernatant. The cells were washed with PBS and centrifuged at 10000 rpm for 1 minute to remove a supernatant. One ml PBS or PBS-T (PBS containing 0.05% Tween 20) containing 1% goat serum was added thereto, and the cells were stirred and subjected to blocking reaction for 10 minutes.

After blocking, the cells were centrifuged at 10000 rpm for 1 minute to remove a supernatant. The first labeled antibody-containing reagent prepared above was added thereto, and the cells were reacted for 30 minutes at room temperature.

After the antibody reaction, the cells were centrifuged at 10000 rpm for 1 minute to remove a supernatant, and the pellet was washed by pipetting in PBS or PBS-T. Thereafter, the cells were centrifuged at 10000 rpm for 1 minute to remove a supernatant, and the cells were washed again with 1 ml PBS or PBS-T and centrifuged at 10000 rpm for 1 minute to remove a supernatant. The cells were suspended in PBS and observed under a fluorescence microscope and measured by flow cytometer FACSCalibur (FSC; E00 1.00, SSC304 v FL1; 340 v FL2; 282 v), and the number of cells for each fluorescence label was counted to prepare a histogram.

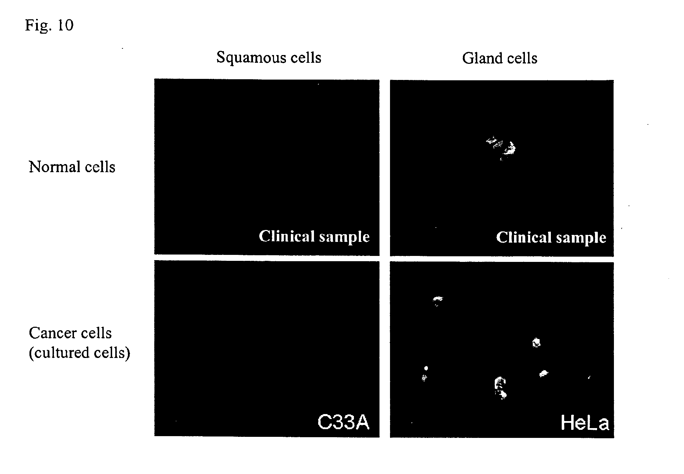

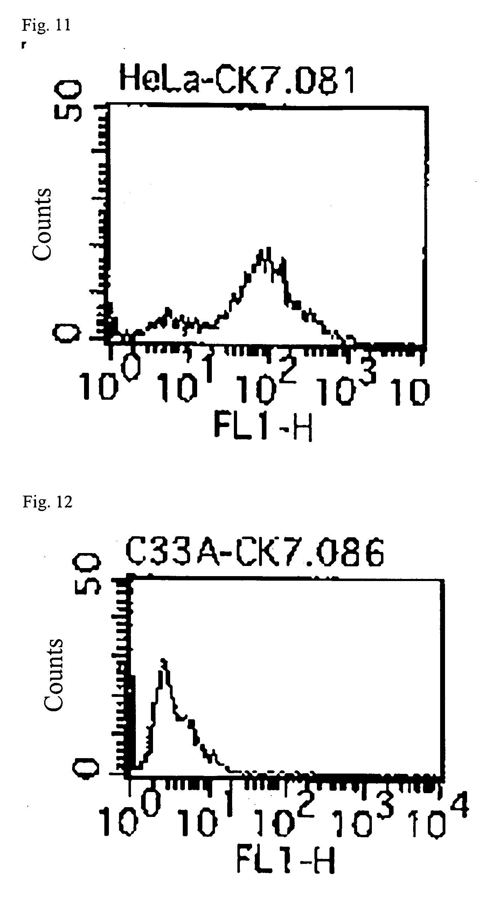

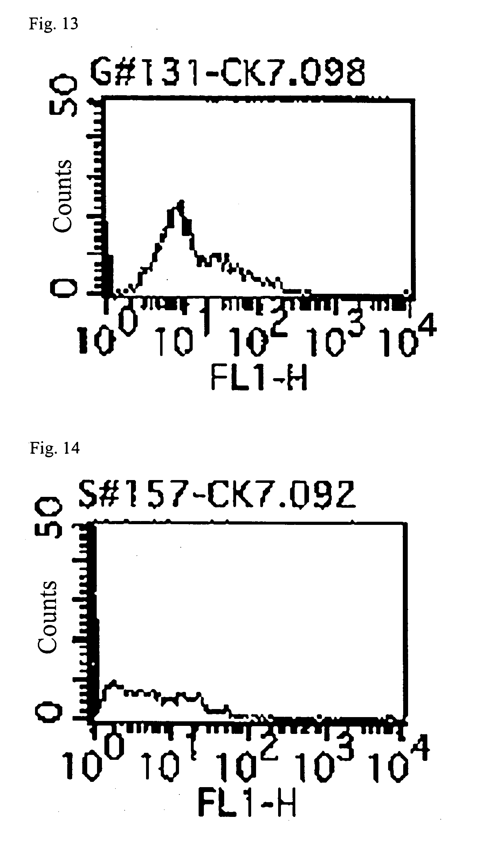

Fig. 10 is a fluorescence microphotograph showing the result of reaction with the HeLa cells, C33A cells, normal gland cells and normal squamous cells. Histograms for each kind of cell, showing the relationship between the green fluorescence intensity derived from the fluorescence label Alexa488 and the number of cells, are shown in Figs. 11 to 14.

The same procedure as above was carried out except that the second labeled antibody-containing reagent and the third labeled antibody-containing reagent were used in place of the first labeled antibody-containing reagent. However, the third labeled antibody-containing reagent was used for only C33A cells and normal squamous cells.

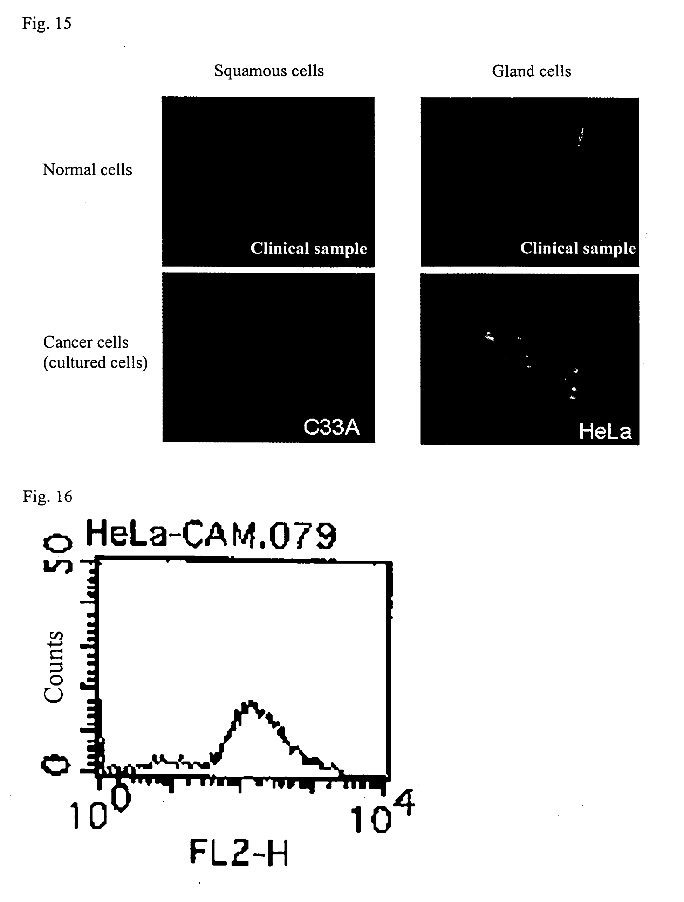

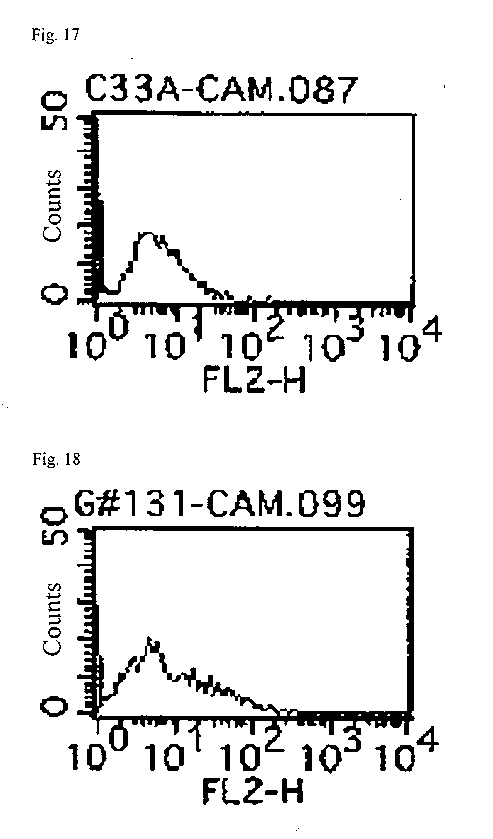

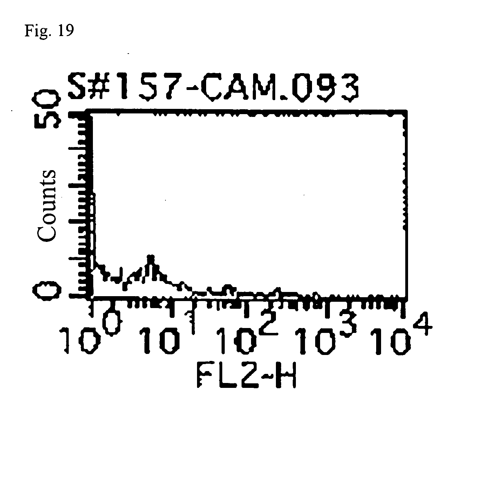

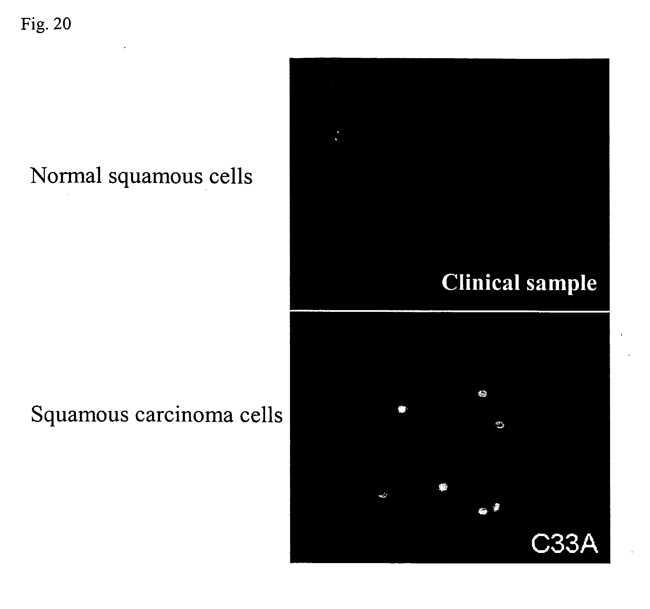

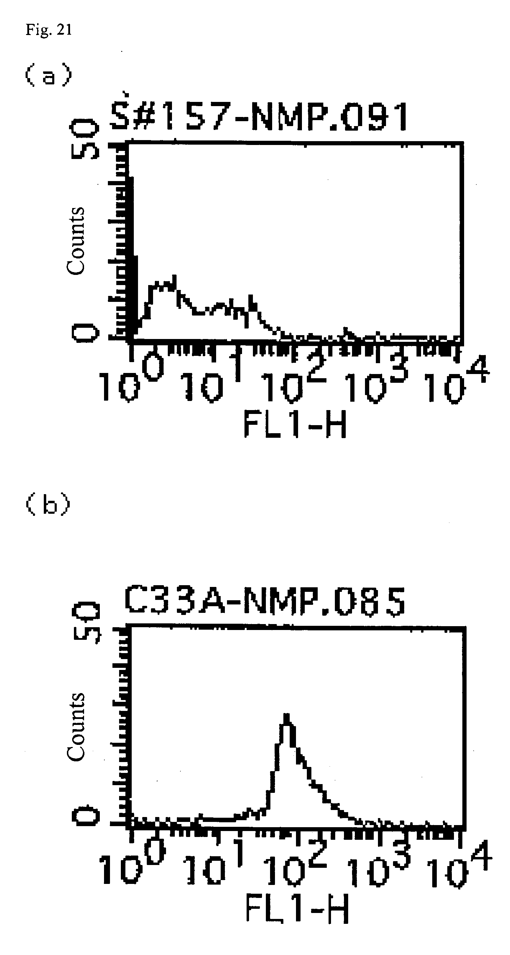

Microphotographs of cells showing the result of reaction with the second labeled antibody-containing reagent are shown in Fig. 15, and histograms showing the relationship between the orange fluorescence intensity derived from R-PE and the number of cells are shown in Figs. 16 to 19. Microphotograph of C33A cells and normal squamous cells showing the result of reaction with the third labeled antibody-containing reagent are shown in Fig. 20, and histograms showing the relationship between the green fluorescence intensity derived from the fluorescence label Alexa488 and the number of cells are shown in Fig. 21. In Fig. 21, (a) is a histogram of normal squamous cells, and (b) is a histogram of squamous carcinoma cells.

### [Staining result of the cells]

In Figs. 10 to 21, a peak with an intensity of about 10² was recognized in histograms where fluorescence was observed in a microphotograph, and the presence of correlation could be confirmed. From Figs. 10 to 14, it could be confirmed that when the first labeled antibody-containing reagent was used, the antibody showed reaction with HeLa cells and normal gland cells and did not show reaction with C33A and normal squamous cells.

From Figs. 15 to 19, it was confirmed that when the second labeled antibody-containing reagent was used, the reaction with HeLa cells was positive, but the reaction with normal gland cells, normal squamous cells and C33A cells was negative.

From Figs. 20 and 21, it could be confirmed that when the third labeled antibody-containing reagent was used, the reaction with C33A cells was positive, but the reaction with normal squamous cells was negative.

Accordingly, it can be understood that gland cells and/or squamous carcinoma cells can be screened by judging each reactivity with the first labeled antibody, the second labeled antibody and the third labeled antibody.

### [Judgment of squamous carcinoma cells by nucleus staining]

### Example 2

About 1×10⁶ epithelial cells in mouth cavity mucosa were introduced into a tube, and 100 µl of 300 µg/ml ribonuclease A (#R-4612 manufactured by Sigma) diluted with PBS was added to the cells to prevent non-specific staining of RNA. The cells were stirred for 5 minutes at room temperature and centrifuged at 10000 rpm for 1 minute to remove a supernatant. Then, 500 µl of 10 µM PI (propidium iodide) was added as a nucleus staining solution thereto, and the cells were stirred for 30 minutes at room temperature. The cells were centrifuged at 10000 rpm for 1 minute to remove a supernatant, and after 500 µl of 0.05% PBST was added thereto, the cells were centrifuged at 10000 rpm for 1 minute. The medium was replaced by a suitable amount of PBS, and the cells were subjected to a flow cytometer having the constitution shown in Fig. 6.

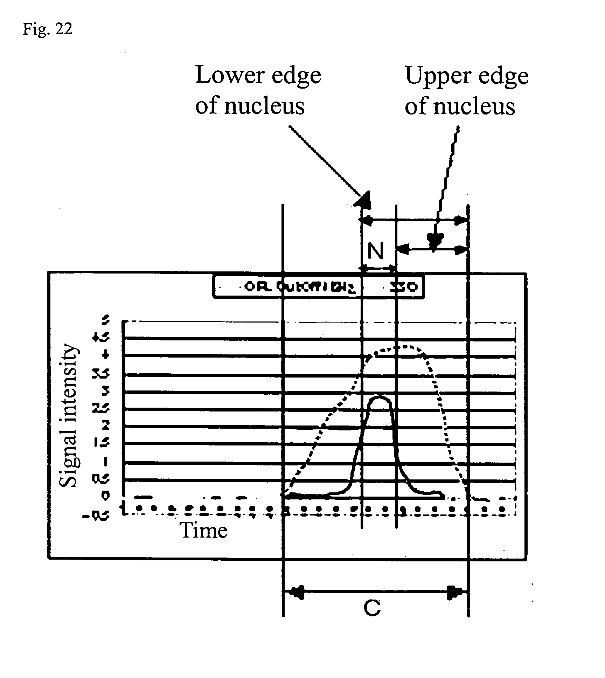

The sample was excited by an argon ion laser at 488 nm, and a fluorescence profile in orange derived from nuclear staining, a side scattered light profile, were recorded, and an image of cells passing through the flow cell was formed. The nucleus staining profile and side scattered light profile are shown in Fig. 22.

In Fig. 22, the solid line is a fluorescence profile derived from nucleus staining, and the broken line is a scattered light profile. The cell size (C) determined from the side scattered light profile agreed almost with the size of cell measured from a cell image. It could be confirmed that the position of nucleus determined from the cell image is correlated with the position of nucleus pulse in the profile in Fig. 22, and it could also be confirmed that the nucleus size (N) corresponds to the nucleus fluorescence pulse (half width value).

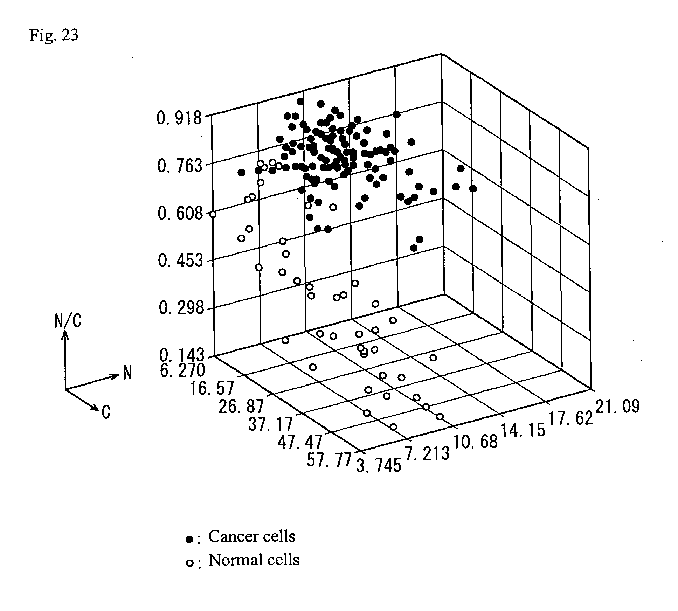

A sample consisting of a mixture of squamous carcinoma cells and squamous normal cells was prepared and subjected to a flow cytometer. A profile corresponding to Fig. 22 was prepared from individual cells passing through the flow cell, and from the cell size (C) and the nucleus size (N) determined from the profile, N/C was calculated. The calculated N, C, and N/C of the measurement sample (cell group) were dot-plotted as X, Y and Z-axis respectively on three-dimensional coordinates, as shown in Fig. 23. In Fig. 23, the squamous carcinoma cells are plotted in black circle "•", while the normal squamous cells are plotted in white circle "○". Generally, squamous carcinoma cells show a high N/C ratio, and thus the plot position of the squamous carcinoma cells is separated from the plot position of the normal squamous cells, as can be seen from the three-dimensional coordinates shown in Fig. 23. It follows that even if judgment by a cell image is not conducted, the ratio of the nucleus size to the cell size (N/C) is calculated from the nucleus staining profile and side scattered light profile according to the flow chart shown in Fig. 7, and when the N/C ratio is higher than a predetermined value, the sample is judged to be cancer cells. In this manner the accuracy of screening of squamous carcinoma cells can further be improved.

### (Measurement of model sample)

### Example 3

Oral mucosal epithelial cells as a substitute for the cervical normal squamous cells, HeLa cells as the cervical adenocarcinoma cells, and C33A cells as the cervical squamous carcinoma cells were used to prepare model samples, that is, a normal sample and 2 abnormal samples (cervical adenocarcinoma cells and cervical squamous carcinoma cells), and whether screening of cervical cancer in these samples was feasible or not was verified.

### (Preparation of the model samples)

As the model sample (model sample of the normal sample) of cervical normal squamous cells, oral mucosal epithelial cells, about 2×10⁵ cells/tube, preserved in a preservative solution PreservCyt (Cytyc; Cat#0234004) were used.

As the model sample of cervical adenocarcinoma cells, about 1×10⁵ HeLa cells (from about 2×10⁵ cells/tube preserved in PreservCyt) were mixed with about 1×10⁵ cells of the above model sample of normal squamous cells, to prepare about 2×10⁵ cells in total per tube.

As the model sample of cervical squamous carcinoma cells, about 1×10⁵ C33A cells (from about 2×10⁵ cells/tube preserved in PreservCyt) were mixed with about 1×10⁵ cells of the above model sample of normal squamous cells, to prepare about 2×10⁵ cells in total per tube.

### (Treatment of removal of mucus)

Each model sample described above was introduced into a 1.5 ml centrifuge tube and centrifuged at 10,000 rpm for 1 min., and the supernatant was discarded. Then, the cells were washed once with PBST (PBS containing 0.05% Tween 20) and centrifuged at 10,000 rpm for 1 min., and the supernatant was discarded, whereby a pellet was prepared.

After 400 µl PBS was added to the tube containing the pellet prepared above, 400 µl of 10% N-acetyl-L-cysteine (Cat#A7250, SIGMA) in PBS was added to the pellet which was then stirred lightly with a voltex mixer, and then 400 µl PBST was added thereto. Then, the sample was centrifuged at 10,000 rpm for 1 min., and the supernatant was discarded. Then, the sample was washed again with 600 µl PBST and centrifuged at 10,000 rpm for 1 min., and the supernatant was discarded. Then, the sample was washed once with 600 µl PBST and centrifuged at 10,000 rpm for 1 min., and the supernatant was discarded, whereby a pellet from which the mucus had been removed was prepared.

### (Treatment of dispersion of the cells)

Four hundred µl Zamboni fixation solution (0.21 % 2,4,6-trinitrophenol [WAKO Cat#205-08672] and 2% paraformaldehyde [Cat#EMS-80, Nacalai Tesque]) was added to the tube containing the above pellet from which the mucus had been removed. The pellet was stirred lightly with a voltex mixer and reacted by a rotator for 10 min., and 400 µl PBST was added thereto, and the sample was centrifuged at 10,000 rpm for 1 min., and the supernatant was discarded. Then, the pellet was washed with 600 µl PBST and then centrifuged at 10,000 rpm for 1 min., and the supernatant was discarded. The pellet was washed again with 600 µl PBST and centrifuged at 10,000 rpm for 1 min., and the supernatant was discarded, and 300 µl PBS was added thereto. After pre-incubation at 37°C for about 5 min., 300 µl enzyme reaction solution (2.4 µl (final concentration 0.2%) of 25% collagenase type I (Cat#4196, Warthington), 2.4 µl (final concentration 0.2%) of 25% collagenase type II (Cat#4176, Warthington), 2.4 µl (final concentration 0.1%) of 12.5% protease (Cat#P-5027, SIGMA), 292.8 µl PBS) was added thereto, and the mixture was reacted at 37°C for 2.5 min. After the reaction, 600 µl of 1% PI reaction stop buffer (10 µl protease inhibitor/1 ml PBS) (protease inhibitor, SIGMA Cat#P8340) previously cooled on ice was added thereto and centrifuged at 10,000 rpm for 1 min., and the supernatant was discarded. The pellet was washed with 600 µl PBST and centrifuged at 10,000 rpm for 1 min., and the supernatant was discarded.

Further, the pellet was suspended with 300 µl PBST and passed through a filter of 100 µm in diameter to remove cell aggregates. The filter was washed with 300 µl PBST, and the wash was recovered. Then, the filtrate and wash were combined and centrifuged at 10,000 rpm for 1 min., and the supernatant was discarded.

### (Preparation of the antibody reaction solution)

A mixture of monoclonal mouse anti-human cytokeratin 7 (Dako Cytomation, Cat#M7081) (final concentration 2.6 µg/ml) and Alexa647-RPE goat anti-mouse IgG (Molecular Probe, Cat#A20990) (final concentration 7.8 µg/ml) diluted with PBST was shielded from light and reacted under stirring with a rotator for 5 min., and mouse IgG was added at a final concentration of 128.8 µg/ml thereto and reacted under stirring with a rotator for 5 min., whereby a first labeled antibody solution was prepared.

A mixture of anti-cytokeratin (CAM5.2) (Becton Dickinson, Cat#349205) (cytokeratin 8 antibody) (final concentration 2.5 µg/ml) and PRE F(ab')₂ goat anti-mouse IgG (Dako Cytomation, Cat#R0480) (final concentration 6.25 µg/ml) diluted with PBST was shielded from light and reacted under stirring with a rotator for 5 min., and mouse IgG was added at a final concentration of 151.2 µg/ml thereto and reacted under stirring with a rotator for 5 min., whereby a second labeled antibody solution was prepared.

A mixture of NMP179 antibody (final concentration 4.89 µg/ml) and Alexa488 F(ab')₂ goat anti-mouse IgG (Molecular Probe, Cat#A11017) (final concentration 10 µg/ml) diluted with PBST was shielded from light and reacted under stirring with a rotator for 5 min., and mouse IgG was added at a final concentration of 246.4 µg/ml thereto and reacted under stirring with a rotator for 5 min., whereby a third labeled antibody solution was prepared.

The first, second and third labeled antibody solutions were mixed in equal amounts to prepare an antibody reaction solution.

### (Antibody reaction)

Six hundred µl of 1% goat serum PBST solution (goat serum: Cedarlanelabs Cat#CL1200) was added to the tube containing the above pellet from which cell aggregates had been removed, and the cells were reacted for 10 minutes under stirring with a rotator and then centrifuged at 10,000 rpm for 1 minute, and the supernatant was discarded. Two hundred µl of the antibody reaction solution was added thereto, and the mixture was shielded from light and reacted for 30 minutes under stirring with a rotator. Then, the mixture was centrifuged at 10,000 rpm for 1 minute to discard a supernatant, then washed with 600 µl PBST and centrifuged at 10,000 rpm for 1 minute, and the supernatant was discarded. The cells were washed again 600 µl PBST and centrifuged at 10,000 rpm for 1 minute, and the supernatant was discarded.

### (Measurement)

Two hundred µl RET-SHEATH (Sysmex; Cat#RSE-900A) was added to the tube containing the above pellet subjected to the antibody reaction, and the cells were suspended and measured for forward scattered light and fluorescence by a flow imaging cytometer having the optical system shown in Fig. 6.

### (Results)

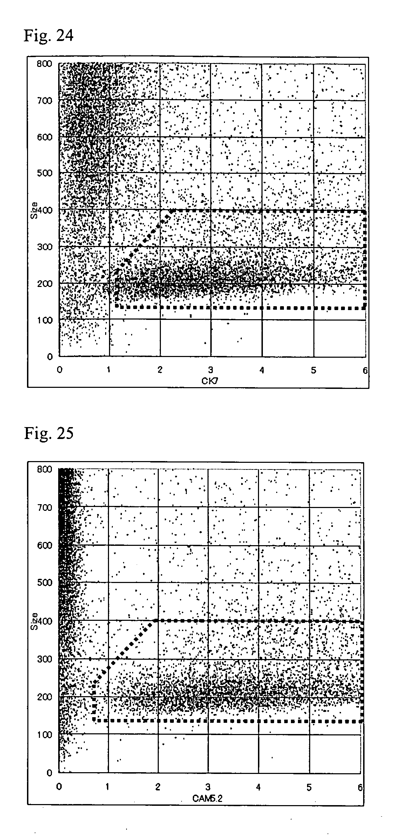

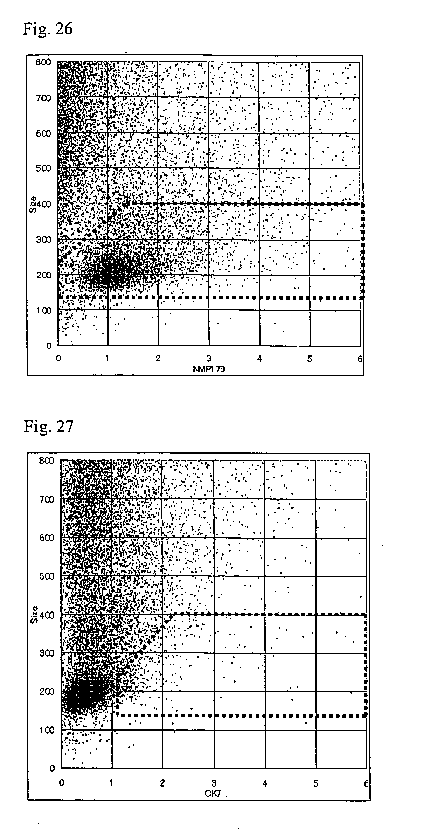

The results of measurement of the model sample of cervical adenocarcinoma cells are shown in Figs. 24 to 26. In each profile, the forward scattered light pulse width is shown on the ordinate, while the fluorescence intensity derived from the first fluorescence labeled antibody is shown on the abscissa in Fig. 24, the fluorescence intensity derived from the second fluorescence labeled antibody on the abscissa in Fig. 25, and the fluorescence intensity derived from the third fluorescence labeled antibody on the abscissa in Fig. 26. Each region enclosed with a broken line indicates a candidate region of cancer or atypical cells (hereinafter referred to as atypical cell candidate region). When a cell population is recognized in each atypical cell candidate region, the reactivity to each labeled antibody can be judged to be positive. Each atypical cell candidate region can be previously determined on the basis of results of measurement of many samples. Cell populations were recognized in atypical cell candidate regions in Figs. 24 to 26, and this model sample could be confirmed to contain cells belonging to group I in Table 1.

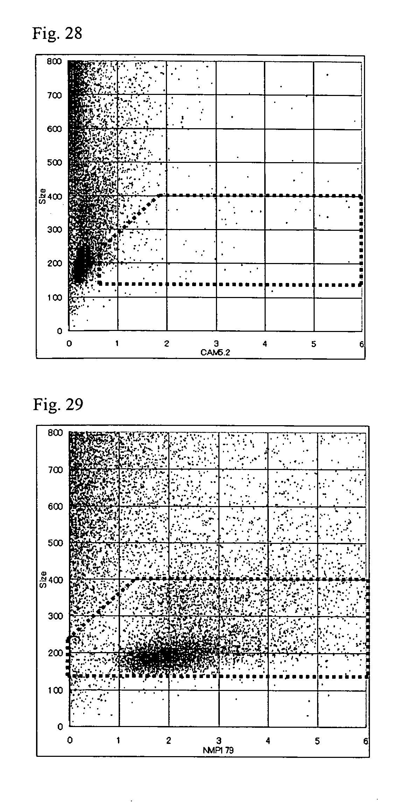

The results of measurement of the model sample of cervical squamous carcinoma cells are shown in Figs. 27 to 29. In each profile, the ordinate has the same meaning as above. The fluorescence intensity derived from the first fluorescence labeled antibody is shown on the abscissa in Fig. 27, the fluorescence intensity derived from the second fluorescence labeled antibody on the abscissa in Fig. 28, and the fluorescence intensity derived from the third fluorescence labeled antibody on the abscissa in Fig. 29. A region enclosed with a broken line has the same meaning as above. No cell population was recognized in atypical cell candidate regions in Figs. 27 and 28, while a cell population was recognized in an atypical cell candidate region in Fig. 29, and this model sample could be confirmed to contain cells belonging to group III in Table 1.

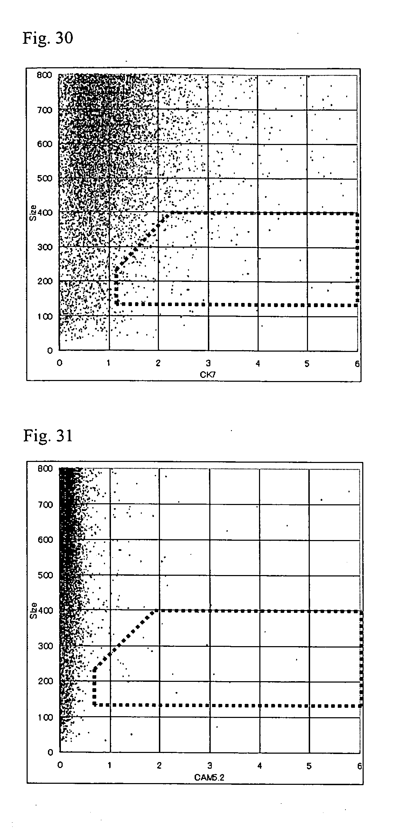

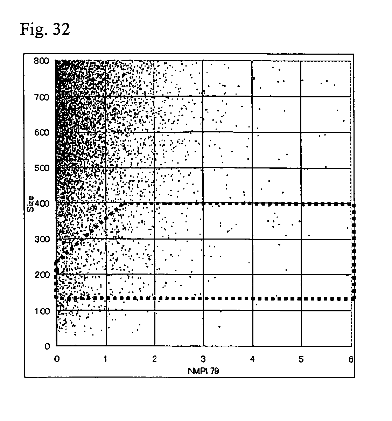

The results of measurement of the model sample of cervical normal squamous cells are shown in Figs. 30 to 32. In each profile, the ordinate has the same meaning as above. The fluorescence intensity derived from the first fluorescence labeled antibody is shown on the abscissa in Fig. 30, the fluorescence intensity derived from the second fluorescence labeled antibody on the abscissa in Fig. 31, and the fluorescence intensity derived from the third fluorescence labeled antibody on the abscissa in Fig. 32. A region enclosed with a broken line has the same meaning as above. No cell population was recognized in atypical cell candidate regions in Figs. 30 to 32, and a cell population was recognized outside of the atypical cell candidate region, and this model sample could be confirmed to contain cells belonging to group IV in Table 1.

## Claims

1. A method for screening cervical cancer, comprising the steps of:
preparing a measurement sample by reacting cervical cells with a first labeled antibody reacting with gland cells, a second labeled antibody reacting with adenocarcinoma cells and a third labeled antibody reacting with atypical cervical squamous cells, the antibodies being labeled with mutually distinguishable labels respectively,
detecting the labels derived from the respective labeled antibodies bound to the cervical cells, and
judging the presence or absence of adenocarcinoma cells and/or atypical squamous cells, on the basis of the detected labels.

2. The method for screening cervical cancer according to claim 1, wherein at least one of the labels is a fluorescence label.

3. The method for screening cervical cancer according to claim 1 or 2, wherein the judging step comprises the steps of:
judging whether the reaction of the cells in the measurement sample with the first labeled antibody is positive or negative on the basis of the detected first label of the first labeled antibody,
judging whether the reaction of the cells judged to be positive to the first labeled antibody with the second labeled antibody is positive or negative on the basis of the detected second label of the second labeled antibody, and
judging whether the reaction of the cells judged to be negative to the first labeled antibody with the third labeled antibody is positive or negative on the basis of the detected third label of the third labeled antibody.

4. A method for screening cervical cancer, comprising the steps of:
preparing a measurement sample by reacting cervical cells with a first fluorescence-labeled antibody reacting with gland cells, a second fluorescence-labeled antibody reacting with adenocarcinoma cells and a third fluorescence-labeled antibody reacting with atypical cervical squamous cells, the antibodies being labeled with mutually distinguishable fluorescences respectively,
irradiating the measurement sample with an exciting light,
detecting the fluorescence emitted by the measurement sample, and
judging the presence or absence of adenocarcinoma cells and/or atypical squamous cells, on the basis of the detected fluorescence.

5. The method for screening cervical cancer according to any of claims 1 to 4, wherein the antibody in the first labeled antibody is at least one selected from the group consisting of MUC1 antibody, cytokeratin 7 antibody, and cytokeratin 18 antibody.

6. The method for screening cervical cancer according to any of claims 1 to 5, wherein the antibody in the second labeled antibody is at least one selected from the group consisting of cytokeratin 8 antibody and HIK1083 antibody.

7. The method for screening cervical cancer according to any of claims 1 to 6, wherein the antibody in the third labeled antibody is at least one selected from the group consisting of NMP 179 antibody, p16^{INK4A} antibody, Ki-67 antibody, p53 antibody, p21 antibody, EMA antibody, CEA antibody and MIB-1 antibody.

8. The method for screening cervical cancer according to any of claims 4 to 7, wherein the judging step comprises steps of:
judging whether the reaction of the cells in the measurement sample with the first fluorescence-labeled antibody is positive or negative by measuring the intensity of the fluorescence derived from the first fluorescence label emitted by the cells,
judging whether the reaction of the cells judged to be positive to the first fluorescence-labeled antibody with the second fluorescence-labeled antibody is positive or negative by measuring the intensity of the fluorescence derived from the second fluorescence label emitted by the cells, and
judging whether the reaction of the cells judged to be negative to the first fluorescence-labeled antibody with the third fluorescence-labeled antibody is positive or negative by measuring the intensity of the fluorescence derived from the third fluorescence label emitted by the cells.

9. The method for screening cervical cancer according to any of claims 4 to 7, comprising the steps of:
allowing the measurement sample to flow through a flow cell of a flow cytometer, and
measuring the fluorescence emitted by the cells for the fluorescence parameter derived from the first fluorescence label, the fluorescence parameter derived from the second fluorescence label, and the fluorescence parameter derived from the third fluorescence label, and wherein the judging step includes step of judging the presence or absence of adenocarcinoma cells and/or atypical squamous cells on the basis of the respective fluorescence parameter values.

10. The method for screening cervical cancer according to claim 9, wherein the fluorescence parameter is selected from the group consisting of fluorescence intensity, fluorescence pulse width and fluorescence pulse area.

11. The method for screening cervical cancer according to claim 9 or 10, wherein the scattered light parameter on the cells in the measurement sample is measured further to judge the presence or absence of adenocarcinoma cells and/or atypical squamous cells.

12. The method for screening cervical cancer according to claim 11, wherein the scattered light parameter is selected from the group consisting of forward scattered light intensity, forward scattered light pulse width and side scattered light pulse width.

13. The method for screening cervical cancer according to claim 11, wherein a two-dimensional scatter diagram having two axes wherein one kind of fluorescence parameter selected from the group consisting of the fluorescence parameter derived from the first fluorescence label, the fluorescence parameter derived from the second fluorescence label and the fluorescence parameter derived from the third fluorescence label is on one axis and the scattered light parameter is on the other axis is prepared to judge the presence or absence of adenocarcinoma cells and/or atypical squamous cells.

14. The method for screening cervical cancer according to claim 9, wherein:
cervical cells in the measurement sample are previously stained with a fluorescent dye capable of staining nuclei, and
a fluorescence profile derived from staining of nuclei of the cervical cells, an emission profile not derived from staining of nuclei but derived from emission from the cervical cells, a forward scattered light profile and a side scattered light profile are compared with one another to judge the presence or absence of adenocarcinoma cells and/or atypical squamous cells.

15. The method for screening cervical cancer according to claim 9 or 10, wherein the flow cytometer has an imaging means, and a cell image is given by imaging the cells judged to be positive in reaction to the third fluorescence-labeled antibody.

16. The method for screening cervical cancer according to any of claims 1 to 15, wherein the cervical cells is a cell population dispersed in single cells.

17. The method for screening cervical cancer according to any of claims 1 to 8, wherein the cervical cells are cells on a smear sample.

18. A diagnostic reagent for cervical cancer, which comprises a first fluorescence-labeled antibody reacting with gland cells, a second fluorescence-labeled antibody reacting with adenocarcinoma cells and a third fluorescence-labeled antibody reacting with atypical cervical squamous cells, the antibodies being labeled with mutually distinguishable fluorescences respectively.

19. The diagnostic reagent according to claim 18, wherein:
the antibody in the first labeled antibody is at least one selected from the group consisting of MUC1 antibody, cytokeratin 7 antibody, and cytokeratin 18 antibody,
the antibody in the second labeled antibody is at least one selected from the group consisting of cytokeratin 8 antibody and HIK1083 antibody, and
the antibody in the third labeled antibody is at least one selected from the group consisting of NMP179 antibody, p16^{INK4A} antibody, Ki-67 antibody, p53 antibody, p21 antibody, EMA antibody, CEA antibody and MIB-1 antibody.

20. A method of automatically diagnosing cervical cancer, wherein in the screening method of claim 1,
the presence or absence of adenocarcinoma cells and/or atypical squamous cells is automatically judged, and
when the ratio of cells judged to be adenocarcinoma cells and/or atypical squamous cells to the cell population contained in the measurement sample is higher than a predetermined value, the cervical cancer is judged to be adenocarcinoma or squamous cell carcinoma.

21. A method for screening cervical cancer, comprising the steps of:
preparing a measurement sample by reacting with cervical cells, an anti-gland cell labeled antibody reacting with gland cells and an anti-atypical squamous cell antibody reacting with atypical cervical squamous cells, the antibodies being labeled with mutually distinguishable labels respectively,
detecting labels derived respectively from the respective labeled antibodies bound to the cervical cells, and
judging the presence or absence of atypical squamous cells, on the basis of the detected labels.

22. The method for screening cervical cancer according to claim 21, wherein the antibody in the labeled antibody reacting with gland cells is at least one selected from the group consisting of MUC1 antibody, cytokeratin 7 antibody, and cytokeratin 18 antibody.

23. The method for screening cervical cancer according to claim 21 or 22, wherein the antibody in the labeled antibody reacting with atypical cervical squamous cells is at least one selected from the group consisting of NMP179 antibody, p16^{INK4A} antibody, Ki-67 antibody, p53 antibody, p21 antibody, EMA antibody, CEA antibody and MIB-1 antibody.

24. An apparatus for screening cervical cells, comprising:
a flow cell for introducing a measurement sample prepared by reacting cervical cells with a first fluorescence-labeled antibody reacting with gland cells, a second fluorescence-labeled antibody reacting with adenocarcinoma cells and a third fluorescence-labeled antibody reacting with atypical cervical squamous cells, the antibodies being labeled with mutually distinguishable fluorescence substances respectively,
a light source for irradiating the cells in the measurement sample flowing in the flow cell with an exciting light,
a first fluorescence detector for detecting the fluorescence derived form the first fluorescence-labeled antibody emitted by the cells irradiated with an exciting light,
a second fluorescence detector for detecting the fluorescence derived form the second fluorescence-labeled antibody emitted by the cells irradiated with an exciting light,
a third fluorescence detector for detecting the fluorescence derived form the third fluorescence-labeled antibody emitted by the cells irradiated with an exciting light, and
an analysis unit for analyzing the detected fluorescence to judge the presence or absence of adenocarcinoma cells and/or atypical squamous cells.

25. The apparatus for screening cervical cells according to claim 24, wherein the apparatus further comprises a light scattered light detector, and the detector further analyzes a detected scattered light.

26. The apparatus for screening cervical cells according to claim 25, wherein the apparatus further comprises an imaging means, and the analysis unit further analyzes an image.
